# EUROPEAN PATENT APPLICATION

(11) **EP 4 670 749 A2**
(43) Date of publication of application: **31.12.2025**
(21) Application number: 25214887.9
(22) Date of filing: 21.02.2020
(51) Int. Cl.: A61L 27/50

(54) **DECELLULARIZED MUSCLE MATRICES AND METHODS FOR MAKING AND USING SAME**

(30) Priority: 21.02.2019 US 201962808759 P
(62) Divisional of application: 20717998.7
(71) Applicant: Musculoskeletal Transplant Foundation, Edison, New Jersey 08837 (US)
(72) Inventor: IMMING, Emily, NJ, 08527 (US); HUANG, Yen-Chen, NJ, 08816 (US); SEMLER, Eric, NJ, 07751 (US); SHIKHANOVICH, Roman, NY, 10312 (US); LONG, Marc, NJ, 07834 (US)
(74) Representative: Meissner Bolte Partnerschaft mbB

(57) **Abstract**

Decellularized muscle matrices are provided for use as implants and grafts to repair, regenerate, supplement, reinforce and replace muscle tissue. The decellularized muscle matrices are derived from muscle tissue having preserved extracellular matrix components, retained muscle-forming potential, and from which immunogenic components have been removed. The decellularized muscle matrices are produced in various physical forms and combinations. Methods for making and using the decellularized muscle matrices are also provided.

## Description

### CROSS-REFERENCE TO RELATED APPLICATIONS

The present application claims the benefit of U.S. Provisional Application No. 62/808,759 filed on February 21, 2020, the entire disclosure of which is hereby incorporated by reference herein.

### FIELD OF THE INVENTION

The present invention relates generally to decellularized muscle matrices and methods for making same. More particularly, the present invention relates to matrices derived from muscle tissue having preserved extracellular matrix components, retained muscle-forming potential, and from which immunogenic components have been removed. The decellularized muscle matrices are useful as implants and grafts to repair, regenerate, replace, supplement, reinforce and form muscle tissue.

### BACKGROUND OF THE INVENTION

It is known that several diseases, traumas, injuries and surgical procedures result in the damage and/or loss of muscle tissue. For example, surgical removal of soft tissue tumors and masses often result in the loss of bulk muscle. Other surgical and cosmetic procedures can, to varying degrees, cause muscle damage or loss which may impair functionality as well as aesthetic appearance. Muscle damage can also result from trauma, such as from blunt force impacts, knife wounds and gunshot injuries. Muscle injuries may also result from sports activity, overuse, strain, injuries, or trauma which result in loss of movement and function. Finally, several diseases, including acute and chronic infection and wasting disease, muscle dystrophy and other genetic diseases, cause significant loss of body tissue, including muscle mass.

It is also known that such conditions can be treated by implantation of allograft or autograft muscle tissue, which has produced varying degrees of muscle healing. Skeletal muscle contains myofibers as well as endogenous nerves, blood vessels and connective tissue. Each of these components may provide endogenous growth factors, cytokines and extracellular matrix proteins which can potentially contribute to the ability of muscle tissue to act as a bioactive matrix that promotes muscle regeneration and restoration of function. The particular tissue processing techniques employed may be important to preserving the bioactivity of the matrix. For example, decellularization of allogeneic tissues may be critical to avoiding an immune response upon implantation. Certain decellularized tissue matrices have been used with some success for regeneration and repair of muscle damaged or lost due, for example, without limitation, to the above-mentioned conditions. The decellularized muscle matrices produced and used according to the methods described herein are believed to promote repair, restoration and regeneration of muscle tissue that has been damaged and/or lost due to the above-mentioned and other conditions, as well as volume restoration and repair or regeneration of muscle tissue for aesthetic and cosmetic reasons unrelated to disease or damage.

### SUMMARY OF THE INVENTION

The invention described and contemplated herein relates to decellularized muscle matrices useful as implants and grafts to repair, regenerate and replace muscle tissue. The decellularized muscle matrices are derived from muscle tissue having preserved extracellular matrix components, retained muscle-forming potential, and from which immunogenic components have been removed. The decellularized muscle matrices are produced in various physical forms and combinations, such as porous and non-porous matrices, putty, shaped or molded forms with or without memory retention, and injectable and/or flowable forms. Methods for making and using the decellularized muscle matrices are also provided.

A composition is provided which comprises a decellularized muscle matrix which provides endogenous muscle-forming potential for muscle repair and regeneration and was derived from unprocessed muscle tissue containing endogenous cytokines and endogenous extracellular proteins, wherein the decellularized muscle matrix retains one or more of the endogenous growth factors, endogenous cytokines, and endogenous extracellular proteins. In some embodiments, the one or more endogenous growth factors are selected from one or more of: vascular endothelial growth factor (VEGF), fibroblast growth factor - basic (FGF-b), and insulin growth factor (IGF). In some embodiments, the endogenous extracellular components include collagen IV, laminin, and fibronectin.

The decellularized muscle tissue matrix may be partially or fully decellularized. The decellularized muscle tissue matrix may have a physical form selected from: strips, chunks, bulk, brick, pieces, elongated elements, sheets, slivers, slices, elongated elements, shavings, particulates, homogenate, etc., and combinations thereof.

In some embodiments, the decellularized muscle tissue matrix is in dried form. In some embodiments, the decellularized muscle tissue matrix is hydrated and moldable. In some embodiments, the composition is dried or partially dried and is shapeable or reshapeable into a different shape after rehydration by combination with one or more fluids such as a carrier, additional component, or both.

The composition may be formed into a predetermined three-dimensional shape. In some embodiments, the three-dimensional shape has been formed using a mold. In some embodiments, the composition is flowable and capable of injection through a syringe.

The composition may further comprise one or more biocompatible carriers, one or more additional components, or both. In some embodiments, the biocompatible carrier is selected from: buffered solutions, glycerol, hyaluronate, polyethylene glycol, stearates, cellulose-derived materials, carboxymethyl cellulose (CMC), hydroxypropyl methyl cellulose (HPMC), and combinations thereof. In some embodiments, the one or more additional components are selected from: polymers, ceramics, materials derived from other tissue types, extracellular matrix material derived from one or more tissue types, endogenous or exogenous cells exogenous growth factors, antibiotics, pharmaceutically active agents, and combinations thereof.

A method for preparing a decellularized muscle tissue matrix is also provided which comprises the steps of: (A) optionally, modifying size, shape, or both, of muscle tissue; (B) rinsing muscle tissue, before, after, or both before and after any other step; (C) performing one or more decellularization soaks; and (D) optionally, disinfecting muscle tissue. In some embodiments, method of Claim 22, further comprising the steps of: (E) formulating a product comprising the decellularized muscle tissue matrix; and (F) optionally, packaging the decellularized muscle matrix or product comprising the decellularized muscle tissue matrix.

In some embodiments, the step of (C) performing one or more decellularization soaks comprises contacting the muscle tissue for a period of decellularizing time with at least one decellularizing solution comprising at least one detergent. In some embodiments, all decellularizing solutions used substantially lack pepsin and pepsin derivatives.

In some embodiments, the method for preparing a decellularized muscle tissue matrix further comprising the step of delipidizing the muscle tissue, for a period of delipidizing time, with at least one delipidizing liquid, before, after, or both before and after, any (C) decellularizing and (D) disinfecting steps. In some embodiments, the delipidizing liquid comprises one or more organic solvents.

A method for providing muscle-forming potential to a recipient is also provided which comprises combining the aforesaid composition, which may be at least partially dried or not, with one or more carriers, one or more additional component, or both, during a medical procedure in which the composition will be implanted into the recipient. In some embodiments, the one or more carriers are selected from: platelet rich plasma, bone marrow aspirate, hyaluronate, amniotic fluid, lipoaspirate and combinations thereof. In some embodiments, the one or more additional components are selected from: polymers, ceramics, materials derived from other tissue types, extracellular matrix material derived from one or more tissue types, exogenous growth factors, cells, antibiotics, and combinations thereof.

A method for repair and regeneration of muscle tissue of a recipient is also provided which comprises implanting the aforesaid composition in or on the recipient. In some embodiments, the decellularized muscle matrix supports differentiation and renewal of muscle progenitor cells. In some embodiments, the recipient has a condition which is treatable by repair and regeneration of muscle tissue and the condition is at least one of: volumetric muscle loss, muscle atrophy, degenerative disease, cardiac disease, cardiac damage, sports injuries, and other injuries.

### DETAILED DESCRIPTION OF THE INVENTION

Detailed embodiments of the present invention are disclosed herein. It should be understood that the disclosed embodiments are merely illustrative of the invention which may be embodied in various forms. In addition, each of the examples given in connection with the various embodiments of the invention is intended to be illustrative, and not restrictive. Further, the figures are not necessarily to scale, and some features may be exaggerated to show details of particular components. In addition, any measurements, specifications and the like shown in the figures are intended to be illustrative, and not restrictive. Therefore, specific structural and functional details disclosed herein are not to be interpreted as limiting, but merely as examples for teaching one skilled in the art to variously employ the present invention.

The terms "graft" and"implant" are used synonymously herein and refer to a tissue or organ transplanted from a donor to a recipient. These terms include, but are not limited to, a tissue or organ transferred from one body site to another in the same individual ("autologous graft"), a tissue or organ transferred between genetically identical individuals or sufficiently immunologically compatible to allow tissue transplant ("syngeneic graft"), a tissue or organ transferred between genetically different members of the same species ("allogeneic graft" or "allograft"), and a tissue or organ transferred between different species ("xenograft"). Thus, the donor and the recipient may each, independently, be a human or non -human species of animal.

Any and all tissues recovered from one or more donors, and products, matrices and extracellular matrices derived therefrom, which are discussed herein may be autogenic, allogenic or xenogenic.

The terms"carrier" and"biocompatible carrier" are used interchangeably herein and refer to a pharmaceutically acceptable inert agent or vehicle for delivering one or more active agents to a subject, and is sometimes referred to as an"excipient." The carrier must be of sufficiently high purity and of sufficiently low toxicity to render it suitable for administration to the subject being treated. The carrier further should maintain, or not interfere with, the stability and bioavailability of an active agent.

The term"buffer" or"buffer solution" as used herein refers to a compound, usually a salt, which, when dissolved in an aqueous medium, serves to maintain the free hydrogen ion concentration of the solution within a certain pH range when hydrogen ions are added or removed from the solution. A salt or solution is said to have a"buffering capacity" or to buffer the solution over such a range, when it provides this function. Generally, a buffer will have adequate buffering capacity over a range that is within ±1 pH unit of its pK.

The term"buffered isotonic solution" as used herein refers to any buffer that is commonly used in biological research. Exemplary buffered isotonic solutions include but are not limited to balanced salt solution (BSS), Hank's balanced salt solution or Hank's buffered salt solution (HBSS), Grey's balanced salt solution, phosphate buffered saline (PBS), tris-buffered saline (TBS), etc. The term"isotonic solution" as used herein refers to a solution whose osmolarity and ion concentrations closely match those within normal cells of the body and the blood. Such solutions are often useful as both rinsing solutions and biocompatible carriers.

The muscle tissue suitable for making the decellularized muscle matrices described and contemplated herein includes all types of muscle tissue recoverable from one or more donors, such as without limitation, skeletal (i.e., striated), cardiac, or smooth muscle. Skeletal muscle is often used due simply to the practical reason that skeletal muscle tissue is most easily and often recoverable from donors, both human and non-human. Accordingly, the decellularized muscle matrices and methods for making them are described hereinafter as being derived from skeletal muscle tissue recovered from one or more donors, however, it is understood by persons of ordinary skill in the relevant art that cardiac and smooth muscle tissues are equally suitable as the starting material for the decellularized muscle matrices described and contemplated herein.

As used herein, the term"decellularized" means that cells and cell components such as membrane and DNA material and other cell components have been removed from the muscle tissue. Furthermore, as used herein, the"decellularized" when used alone includes fully and partially decellularized, where"fully decellularized" muscle tissue contains less than about 50 nanograms (ng) of nuclear (DNA) material per milligram (mg) decellularized muscle tissue, and "partially decellularized" muscle tissue contains less than 50% by weight of the total endogenous nuclear (DNA) material originally present in the unprocessed source muscle tissue.

The terms"delipidized" and"delipidizing" are used herein to mean the characteristic and process by which at least a portion of the lipids naturally present in a tissue are removed from the tissue.

As used herein, the term"muscle tissue" means a soft tissue found in most animals which function is to produce force and motion. Generally, there are three types of muscle tissue: (1) cardiac, which is responsible for contraction of the heart, (2) skeletal, which is responsible for moving extremities and external areas of the body, and (3) smooth, which is responsible for contraction of the blood vessels and bowels."Muscle" tissue is used herein to mean one or more of these types of muscle tissue, unless specified otherwise.

As used herein, the term"muscle fiber" and its plural form"muscle fibers" mean naturally occurring fibers which are contained in muscle tissue and comprise myofibers, fascicles, and/or portions thereof. Muscle fibers are not the same as, and should not be confused with muscle slivers, a tissue form which will be described hereinbelow and which are typically comprised of muscle fibers and/or portions thereof.

As used herein, the terms"myofiber" and"myocyte" are used interchangeably and mean the large, highly specialized cells which are the basic cellular unit of skeletal muscle tissue, which also contains extracellular matrix material and other components. Myofibers often have multiple nucleii and range in size from less than 100 to a few hundred microns (um) in diameter and have lengths of from a few millimeters to a few centimeters. Myofibers are bundled together into "fascicles," which are typically sheathed in connective tissue."Myofibrils" are different from myofibers. Myofibrils are basic rod-like units of myofibers and myofibers typically contain many chains of myofibrils. Decellularization is performed to remove myofibers and other cells, as well as components of myofibers and other cells (e.g., DNA, cytoplasm, membrane material, etc.) from muscle tissue.

As used herein, the term"myoblast" means an embryonic cell that becomes a cell of muscle fiber, or myocyte.

As used herein, the terms"myogenic" and"myogenesis" mean the ability (potential) and process, respectively, of forming new muscle tissue, such as by myoblasts or other muscle-forming cell lineages.

As used herein, the terms"myoinductive" and"myoinduction" mean the ability (potential) and process, respectively, by which primitive, undifferentiated and pluripotent cells are stimulated, such as by soluble factors, extracellular matrix, etc., to develop into a muscle-forming cell lineage. For example, a myoinductive factor is one that stimulates primitive, undifferentiated and pluripotent cells to develop into a muscle-forming cell lineage.

As used herein, the terms"myoconductive" and"myoconduction" mean the ability (potential) and process, respectively, by which muscle-forming cells are directed, such as by the texture, topology, roughness, physical properties of a matrix or scaffold, to facilitate and promote formation of muscle tissue by the muscle-forming cells. For example, a myoconductive matrix is one that facilitates and promotes muscle tissue formation on its surface and within the matrix by muscle-forming cells on, in or proximate to the myoconductive matrix.

As used herein, the term"muscle-forming potential" is the property or ability of a compound, substance, material, matrix, or combination thereof, to stimulate, facilitate or promote one or more of myogenesis, myoinduction and myoconduction, which includes the formation of new muscle tissue as well as the repair and reconstruction of existing muscle tissue.

The decellularized muscle matrices are derived from muscle tissue (also referred to herein as"starting muscle tissue,""unprocessed muscle tissue," and"original muscle tissue""), which has been at least partially decellularized, has preserved extracellular matrix components, and retained endogenous muscle-forming potential, including one or more of myogenesis, myoinduction and myoconduction. The extracellular components include, without limitation, collagen IV, laminin, and fibronectin. The retained endogenous growth factors include without limitation, one or more of: vascular endothelial growth factor (VEGF), fibroblast growth factor -basic (FGF-b), as well as insulin growth factor (IGF). Additionally, at least a portion of the endogenous immunogenic components have been removed from the matrices. Immunogenic components endogenous to the muscle tissue, at least a portion of which have been removed, include, without limitation, myofibers and deoxyribonucleic acid (DNA). When describing the size of muscle tissue and decellularized muscle matrices, the dimensional terms"length,""width,'' and"thickness" have their generally accepted meanings and, for any given piece of muscle tissue or decellularized muscle matrix in which the length, width and thickness are not equal to one another, the"length" should be the greatest dimension, the"width" is the next greatest dimension, and the"thickness" should be the least dimension. Nonetheless, in some embodiments, two or more of the length, width and thickness may be about equal to one another. Where there is no specific range or value for the thickness of a particular piece or shape, then the thickness should be understood to be any value within any previously stated range for that piece or shape.

The decellularized muscle matrices provide suitable scaffolds for treatment of muscle damage, injury, degeneration, trauma and/or loss. Successful muscle repair and regeneration scaffolds will provide mechanical stability, support differentiation and renewal of muscle progenitor cells (satellite cells), support peripheral nerve re-innervation and re-vascularization, promote muscle repair by supporting and enabling muscle tissue generation. Additionally, successful muscle repair and regeneration scaffolds will often also facilitate contouring, sculpting or other cosmetic procedures. The decellularized muscle matrices may also be used to fill muscle gaps, defects, deficits, and/or deficiencies.

The decellularized muscle matrices may be produced in various forms and sizes, all of which provide suitable scaffolds for muscle repair and regeneration. All forms and sizes of the decellularized muscle matrices may include whole and/or portions of the naturally occurring muscle fibers of the original muscle tissue from which they are derived. Such muscle fibers, whole as well as portions thereof retain at least a portion of their original structure, function and biological activity of the original. The decellularized muscle matrices described herein may have any of various physical forms (i.e., shapes and sizes) based on their particular intended use and location of implantation in a recipient (patient), including without limitation; particulate, powder, gel, paste, sponge, slurry, sliver, slice, shaving, elongated with or without branches, sheet, sphere, irregular spheres, symmetric or asymmetric pieces, irregularly shaped pieces, monolith, brick, pebbles, etc., as will be recognized by persons of ordinary skill in the relevant art.

For example, as will be described in further detail hereinafter, in some embodiments, the decellularized muscle matrices may be uncut and as-recovered from donors, or in other bulk form, such as, without limitation, pieces, chunks, slices, sheets, blocks, etc., either dry (e.g., lyophilized, air-dried, etc.) or hydrated with one or more hydrating biocompatible carriers, and which are contemplated as being useful for replacement of lost muscle mass. In other embodiments, as will also be described hereinafter, the decellularized muscle matrices may be in particulate form and may be dry (e.g., lyophilized, air-dried, etc.), hydrated and moldable (manual or otherwise), or combined with a carrier and flowable for injection. Hydrated and flowable particulate forms of the decellularized muscle matrices may be combined with one or more hydrating biocompatible carriers. One or more embodiments of the decellularized muscle matrices described herein are believed to be beneficial for recipients (patients) having a condition which is treatable by repair and regeneration of muscle tissue, such as without limitation, at least one of: volumetric muscle loss, muscle atrophy, degenerative disease (e.g., muscular dystrophy), cardiac condition (heart disease, damage to cardiac muscles, pericardium, etc.), and sports or other injuries (sprains, tears, and damage or other trauma to joints, neck, back, limbs, face, etc.).

In still other embodiments, which will be described in further detail hereinbelow, the decellularized skeletal matrices may be in the form of thin slivers of decellularized muscle tissue, such as elongated elements having fiber-like structures, which are different from and not to be confused with the naturally-occurring myofibers, fascicles or muscle fibers of muscle tissue, as described above. Like the particulate forms, decellularized muscle matrices in the form of slivers may be dry (e.g., lyophilized, air-dried, etc.), hydrated and moldable (manual or otherwise), or combined with a carrier and flowable for injection. Smaller sizes and shapes of muscle tissue, as well as muscle fibers, such as without limitation, particulates and slivers, are more efficiently and more quickly decellularized compared to larger sizes and shapes of muscle tissue, such as without limitation, uncut or other bulk form. Furthermore, the particulate and sliver forms of decellularized muscle matrices may be dried (e.g., lyophilized, air-dried, etc.) in a particular predetermined shape, or simply in a mass of indeterminate shape, and may then rehydrated with a biocompatible carrier. Furthermore, the particulate and sliver forms of the decellularized muscle matrices may be lyophilized into a sponge-like mass. As used herein, the terms"sponge" and sponge-like" are used interchangeably to describe certain embodiments of decellularized muscle matrices which, after drying, such as lyophilization (as described below), have a defined dried shape and are porous, elastic, flexible and foamy, which means they have some shape retention and/or shape memory compared to brittle or hard matrices. Sponge and sponge-like decellularized muscle matrices tend to retain and/or return to their defined dried shape upon application of compressing, bending, folding or flexing forces.

The decellularized muscle matrices may, for example, be used as bulking agents for muscle deficiency, muscle damage, and/or muscle loss (e.g., traumatic, surgical (e.g., flaps, oncological resection), acute, or atrophy), as well as injectable forms to treat acute muscle injuries. Additionally, the decellularized muscle matrices may also be used to treat volumetric muscle loss (VML), such as due to muscle trauma, as well as to treat muscle tears and muscle atrophy. Generally, volumetric muscle loss (VML) refers to a skeletal muscle injury in which more than 20% of a muscles volume is lost to traumatic injury. The decellularized muscle matrices are also useful as contouring, sculpting or bulking agents for performing cosmetic or reconstructive surgery to treat muscle loss, damage or injury, as well as for aesthetic reasons and reconstruction and cosmetic procedures. Decellularized muscle matrices may be used in an injectable form to treat small muscle deficiencies. For example, without limitation, the decellularized muscle matrices are suitable for beneficial use in various medical procedures including: body contouring (e.g., calves, biceps, abdominal, etc.), cardiac treatment, surgical reconstruction (including flaps), plastic reconstruction (including breast reconstruction and augmentation), muscle augmentation, muscle repair towards returning to sports and function following sports-related muscle injuries and trauma, surgical repair and reconstruction of general traumatic injuries and loss, military-related body injuries and muscle damage, and genetic degenerative disease.

Several embodiments of suitable methods for making decellularized muscle matrices will now be described. Generally, such methods comprise the steps of: (A) one or more size and/or shape modification steps, (B) one or more rinsing steps, which may be performed before or after any of the other steps, as well as both before and after any of the other steps, (C) one or more decellularization steps (sometimes referred to as"soaks"), (D) one or more disinfection steps, (E) one or more product formulation steps, and (F) packaging. In various embodiments of the disclosed method, some of the aforesaid steps may be omitted, the order of the steps may be varied, or additional steps may be provided. The"muscle tissue" of the method may be the starting muscle tissue in the form isolated from the donor, rinsed muscle tissue, decellularized muscle tissue, disinfected muscle tissue, formulated muscle tissue, packaged soft tissue, delipidized muscle tissue, or sterilized muscle tissue. Embodiments of the disclosed method are discussed further hereinbelow, and exemplary embodiments are presented.

Starting or unprocessed muscle tissue suitable for use to make the decellularized muscle matrices are typically obtained from one or more individual donors and may be fresh or stored frozen (e.g., at temperatures at or below zero °C). In some embodiments, the starting muscle tissue is separated from other tissue types, with blood and other debris removed and separated. However, as will be determinable by persons of ordinary skill in the relevant art, additional cleaning and rinsing steps may be warranted depending on the condition of the starting muscle tissue prior to commencement of the methods described below. Furthermore, in some embodiments, the muscle tissue recovered from one or more donors is not completely separated from other tissue types such that the starting muscle tissue includes at least some quantity of other tissue types such as, without limitation, adipose, nerve, tendon, blood vessel, fascia, other connective tissue, and combinations thereof. The starting muscle should comprise at least 50% by weight of muscle tissue, based on the total weight of the starting muscle tissue. The starting muscle tissue may be autogenic, allogenic, xenogenic, or some combination thereof, Additionally, the donors may be human or non-human animals, including without limitation, porcine, bovine, rodent, equine, canine, feline, primate, etc. When stored frozen, starting muscle tissue is typically, but not necessarily, thawed prior to commencement of further processing steps as described below, depending upon the techniques employed for the (A) one or more size and/or shape modification step(s).

The (A) size and/or shape modification steps are generally, but not necessarily, applied to unprocessed muscle tissue and may be accomplished by any suitable technique, known now or in the future, to persons of ordinary skill in the relevant art, including but not limited to: cutting, slicing, grating, grinding, mincing, homogenizing, etc., to obtain the desired sizes and shapes of muscle tissue depending on the intended final form (i.e., bulk, particulate, slivers, slices, etc.) of the decellularized muscle matrices. For example, without limitation, the starting muscle tissue may be formed into strips, chunks, sheets, larger pieces, smaller pieces, slivers, slices, shavings, elongated elements with or without branches, particulates (symmetric or irregular), etc.

In various embodiments, the (A) one or more size and/or shape modification steps may be performed at one or more temperatures between and including from about -80 °C to about 40 °C, such as at about 25 °C, or at about ambient temperature or higher, or from about 4 °C to about 10 °C, or at a physiological temperature of a living animal, or at about 37 °C. Additionally, the (A) one or more size and/or shape modification steps may be performed at the same or different temperatures from one another.

In some embodiments, the starting muscle tissue may be left intact or initially cut or sliced into relatively large sizes and shapes, such as without limitation, roughly rectilinear, chunk, brick or sheet-like in shape and having, independently, length, width and thickness of from about 20 cm to about 1 cm, such as about 20 cm, x 20 cm x 10 cm, or about 10 cm x 10 cm x 10cm, or about 10 cm x 10 cm x 5 cm, or about 5 cm x 5 cm x 5 cm, or even smaller. Alternatively, or in addition to the foregoing treatment, the muscle tissue may be subjected size and/or shape modification steps which form smaller pieces, slivers or slices. In some embodiments, the muscle tissue can be cut into thin strips, for example without limitation, roughly rectilinear or brick-like shapes and having, independently, length, width and thickness of from about 0.5 cm to about 2 cm, such as about 2 cm x 2 cm x 2cm, or about 2 cm x 2cm x 1cm, or about 2 cm x 1 cm x 1cm, etc. For example, in some embodiments, the muscle tissue may be placed muscle onto a cutting board and the mid-section of the skeletal muscle tissue cut along the direction of the fibers which form the muscle tissue to form slivers of muscle tissue, keeping the ends attached.

In some embodiments, the starting muscle tissue may, either with or without initial cutting or slicing steps as described above for forming pieces (e.g., roughly rectilinear, chunk, brick, sheet like, sliver or slice shapes), be subjected to size and/or shape modification steps which form smaller pieces such as slivers, slices, shavings, elongated elements with or without branches, and particulates. Such size and/or shape modification steps may, for example without limitation, be performed by cutting, milling (including freezer milling), grating (such as, for example, with frozen muscle tissue), grinding, etc. In some embodiments, one or more size and/or shape modification steps are performed after one or more processing steps (such as decellularizing, etc. as described hereinbelow).

In some embodiments, the resulting smaller slices or slivers of muscle tissue may have roughly rectilinear or small brick-like shapes and, independently, length, width and thickness of from about 0.1 cm to about 4 cm, such as about 2 cm x 2 cm x 0.5 cm, or about 1 cm x 2 cm x 1 cm, or about 0.5 cm x 2 cm x 0.5 cm, or about 0.5 cm x 2 cm x 0.25 cm, , or about 0.5 cm x 0.5 cm x 0.5 cm, or about 0.5 cm x 0.5 cm x 0.25 cm, or about 0.5 cm x 0.5 cm x 0.1 cm. In some embodiments, the slices of muscle tissue each have a length and a width of about 0.5 cm x 0.5 cm. In some embodiments, the slivers of muscle tissue each have a length and a width of about 2 cm x 0.5 cm.

In some embodiments, particulates of muscle tissue resulting from one or more of the above-described size and/or shape modification steps have roughly spherical or irregular shapes and average particle size of from about 10 microns (um) to about 5 millimeters (mm), or any range or size therebetween. For example, particles of muscle tissue may, without limitation, have an average particle size of from about 10 um to about 4 mm, or from about 10 um to about 3 mm, or from about 10 um to about 2 mm, or from about 10 um to about 1 mm, or from about 10 um to about 800 um, or from about 25 um to about 750 um, or from about 25 microns to about 500 microns, or from about 25 um to about 250 um, or from about 25 um to about 150 um. For example, without limitation, particles of muscle tissue may have an average particle size of no greater than about 5 mm, or no greater than about 3 mm, or no greater than about 2 mm, or no greater than about 1 mm, or no greater than about 800 um, or no greater than about 750 um, or no greater than about 500 um, or no greater than about 250 um, or no greater than about 200, um, or no greater than about 150 um, or no greater than about 100 um , or no greater than about 75 um, or no greater than about 50 um. In some embodiments, where the decellularized muscle matrix is in particle form, the average aspect ratio (i.e., ratio of length to width, or the two greatest of each particles three dimensions) of the matrix is about 3 or greater.

In some embodiments, slivers or elongated elements of muscle tissue resulting from one or more of the above-described size and/or shape modification steps have roughly relatively elongated shapes, each of which has a length of at least about 2 mm, a width of from about 10 um to about 0.5 cm, an aspect ratio of length to width greater than about 3, and a thickness which is no greater than its width. In a population of slivers or elongated elements, it is not necessary for every individual sliver or elongated element to have the foregoing dimensions, as long as at least some do. In some embodiments, individual slivers or elongated elements of muscle tissue have a length of at least about 2 cm, a width of from about 10 um to about 0.5 cm, an aspect ratio of length to width greater than about 5, and a thickness which is no greater than its width.

Before and after any of the above-described size and/or shape modification steps, the pieces of muscle tissue may be kept in deionized (DI) water to prevent or minimize drying out before and after cutting, milling, etc. In some embodiments, where smaller pieces of muscle tissue are formed during the one or more size and/or shape modification steps, such as without limitation, pieces of length and width of about 2 cm x 2 cm, each such smaller piece is individually weighed to determine the required amount of solution (e.g, DI water or buffered saline solution) necessary to ensure sufficient hydration. In some embodiments, the required amount of solution is, for example without limitation, about 125 milliliters (mL) of solution per gram (g) of muscle tissue, or about 500 ml of solution per 4 g of muscle tissue. When appropriate, smaller sample pieces (e.g., length and width of about 2 cm x 2 cm) may be placed into suitably sized culture flasks with solution, for example without limitation, a single sample piece in a 1 liter culture flask, or two such smaller sample pieces in a 2 liter culture flask.

The (A) one or more size and/or shape modification steps are each performed for a period of time sufficient to accomplish the size and/or shape modification desired. It is well within the ability of persons of ordinary skill in the relevant art to determine such periods of time based on the desired resulting size and shape of the muscle tissue. For example, such periods of time may be from about 2 seconds to about 36 hours, or any time range or value therebetween such as without limitation, from about 15 seconds to about 24 hours, or from about 1 minute to 3 hours. Additionally, the (A) one or more size and/or shape modification steps may each be performed with or without suitable agitation, stirring, vacuum, pressure cycling (i.e., alternating addition and removal of pressure), sonication, perfusion, mixing, or revolutions per minute (rpm), as appropriate and determinable by persons of ordinary skill in the relevant art depending upon the type of size and/or shape modification being performed, the apparatus being used and the degree of size and/or shape reduction desired.

During methods for making the decellularized muscle matrices, after the (A) one or more size and/or shape modification steps are performed, the muscle tissue (i.e., pieces, sheets, slices, slivers, elongated elements, particulates, etc.) is subjected to chemical treatment steps (i.e., (B) one or more rinsing steps, (C) one or more decellularization steps, (D) one or more disinfection steps, and (E) one or more product formulation steps) by sequentially contacting the muscle tissue with several chemical solutions, including without limitation, one or more each of: rinsing solutions, decellularizing solutions, and disinfecting solutions. In some embodiments, the muscle tissue is rinsed with DI water in between two or more of chemical treatment steps.

The (B) one or more rinsing steps may be performed using a rinsing solution comprising at least one of: DI water and a buffered solution. In some embodiments, the rinsing solution comprises DI water. In some embodiments, the (B) one or more rinsing steps may be performed using more than one type of rinsing solution. The (B) one or more rinsing steps may be performed before or after, or both before and after, any of the (C) one or more decellularization steps and any of the (D) one or more disinfection steps, and each such (B) one or more rinsing steps may be performed using the same or different rinsing solutions than the other (B) one or more rising steps.

The (B) one or more rinsing steps are each performed for a period of time sufficient to accomplish the degree of rinsing (i.e., sufficient removal of whatever components, solutions or other substances) desired. It is well within the ability of persons of ordinary skill in the relevant art to determine such periods of time based on the type of rinsing solution used and the desired resulting degree of rinsing to be accomplished. For example, such periods of time may be from about 1 minute to about 2 hours, or any time range or value therebetween such as without limitation, from about 15 minutes to about 1 hour, or from about 20 minutes to about 40 minutes. Additionally, the (B) one or more rinsing steps may each be performed with or without suitable agitation, stirring, vacuum, pressure cycling (i.e., alternating addition and removal of pressure), sonication, perfusion, mixing, or revolutions per minute (rpm), as appropriate and determinable by persons of ordinary skill in the relevant art depending upon the apparatus being used and the degree of rinsing desired.

The (C) one or more decellularization steps may be performed using a decellularizing solution comprising at least one detergent. The (C) one or more decellularization steps may also be referred to herein as"soaks" and facilitate contact and infiltration of the decellularizing solution with the muscle tissue (to distinguish from the (B) one or more rinsing steps (i.e.,"rinsing" or "rinses") which are performed to remove substances such as decellularizing and disinfecting solutions). Suitable detergents include, without limitation, sodium deoxycholate, sodium dodecyl sulfate (SDS), phenyl methyl sulfonyl fluoride (PMSF), TRITON X-100^{®} (a tetramethylbuyl-phenyl- and polyethylene oxide-containing surfactant commercially available from Dow Chemical Company of Midland, Michigan, U.S.A.), and polyoxyethylenesorbitan monooleate, (i.e., polysorbate-80). In some embodiments, one type of decellularizing solution may be used to perform each of the (C) one or more decellularization steps. In some embodiments, more than one decellularizing solution may be used to perform the (C) one or more decellularization steps. For example without limitation, each decellularization step may be performed using a different decellularizing solution.

In some embodiments, more than one decellularization step is performed, each of which may involve use of the same or different decellularizing solution than one or more of the other decellularization steps. In some embodiments, the (C) one or more decellularization steps include three separate decellularization steps (performed sequentially as follows or not), a first of which involves use of a first decellularizing solution comprising tris-buffered saline and PMSF, a second of which involves use of a second decellularizing solution comprising SDS, and a third of which involves use of a third decellularizing solution comprising sodium deoxycholate. In some embodiments the (C) one or more decellularization steps include two separate decellularization steps (performed sequentially as follows or not), a first of which involves use of a first decellularizing solution comprising SDS, and a second of which involves use of a second decellularizing solution comprising sodium deoxycholate.

It is noted that the methods employed by others to make other types of decellularized muscle matrices often use pepsin or a solution comprising pepsin as the decellularizing solution. The methods described and contemplated herein do not use pepsin and the decellularizing solutions used in accordance with the methods described herein do not include pepsin (i.e., suitable decellularizing solutions substantially lack pepsin or pepsin derivatives). This is believed to minimize unnecessary disruption or breaking up of the muscle tissue components and, thereby, better maintain the integrity of the extracellular matrix, endogenous proteins and growth factors of the starting muscle tissue during the (C) one or more decellularization steps.

In some embodiments, regardless of the size and shape of the final decellularized muscle matrices (e.g., bulk, slices, slivers, elongated elements, particulates, etc.), the decellularized muscle matrices may be "fully decellularized," which mean that DNA and myofibers have been removed such that the decellularized muscle matrices contain less than about 50 ng of nuclear (DNA) material per mg of decellularized muscle matrices. There are several methods for measuring the degree of decellularization, i.e., the DNA content of decellularized muscle matrices, however, the aforesaid "fully decellularized" condition of the decellularized muscle matrices which contain less than about 50 ng of nuclear (DNA) material per mg of decellularized muscle matrices are determined according to the test method described in Example 1 provided hereinbelow. In some embodiments, again regardless of the size and shape of the final decellularized muscle matrices, the decellularized muscle matrices may be partially decellularized, wherein only DNA has been removed and at least a portion of the endogenously present myofiber has been retained (which retains more volume of such matrices). Degree of decellularization may also be assessed by measuring the quantity of myofiber remaining in the decellularized muscle matrices.

The (C) one or more decellularization steps are each performed for a period of time sufficient to accomplish the degree of decellularization (i.e., full or partial) desired. It is well within the ability of persons of ordinary skill in the relevant art to determine such periods of time based on the type of decellularizing solution used and the desired resulting degree of decellularization. For example, such periods of time may be from about 1 minute to about 7 days, or any time range or value therebetween such as without limitation, or from about 1 minute to about 5 days, or from about 1 minute to about 2 days, or from about 1 minute to about 24 hours, or from about 1 minute to about 6 hours, or from about 5 minutes to about 6 hours, or from about 5 minutes to about 2 hours, or from about 5 minutes to about 60 minutes, or from about 10 minutes to about 60 minutes, or from about 15 minutes to about 40 minutes. Additionally, the (C) one or more decellularization steps may each be performed with or without suitable agitation, stirring, vacuum, pressure cycling (i.e., alternating addition and removal of pressure), sonication, perfusion, mixing, or revolutions per minute (rpm), as appropriate and determinable by persons of ordinary skill in the relevant art depending upon the apparatus being used and the degree of decellularization desired.

In various embodiments, the (C) one or more decellularization steps may be performed at one or more temperatures between and including from about 2 °C to about 45 °C, such as about 4 °C to about 40 °C, or about 40 °C or less, or about 25 °C or less, or at about ambient temperature, or from about 15 °C to about 25 °C, or from about 18 °C to about 22 °C. Additionally, the (C) one or more decellularization steps may be performed at the same or different temperatures from one another.

The (D) one or more disinfection steps may be performed using a disinfection solution comprising at least one disinfectant. Suitable disinfectants include one or more of: acetic acid, peracetic acid, ethanol, propanol, hexane, hydrogen peroxide, one or more antibiotics and combinations thereof. Suitable antibiotics include, without limitation, erythromycin, bacitracin, triclosan, neomycin, penicillin, amphotericin B, vancomycin, primaxin, polymyxin B, tetracycline, viomycin, chloromycetin and streptomycin, cefazolin, ampicillin, azactam, tobramycin, clindamycin and gentamycin. In some embodiments, more than one disinfecting solution may be used to perform the (D) one or more disinfection steps. For example without limitation, each disinfection step may be performed using a different type of disinfecting solution. In some embodiments, one type of disinfecting solution may be used to perform each of the (D) one or more disinfection steps. In some embodiments, only one disinfecting step is performed and the disinfecting solution comprises peracetic acid. In some embodiments, more than one disinfection step is performed, each of which may involve use of the same or different disinfecting solution than one or more of the other disinfection steps. In some embodiments, the (D) one or more disinfection steps include two separate disinfection steps (performed sequentially as follows or not), a first of which involves use of a first disinfecting solution comprising peracetic acid, a second of which involves use of a second disinfecting solution comprising one or more antibiotics. In some embodiments, where the disinfecting solution comprises peracetic acid, the peracetic acid may, for example without limitation, have a concentration of from about 0.1 % to about 1.0 % by weight based on the total weight of the peracetic acid, or any concentration therebetween, such as from about 0.2 % to about 1.0 %, or from about 0.5 % to about 0.8 %, or from about 0.2 % to about 0.4%.

The (D) one or more disinfection steps may each be performed for a period of time sufficient to accomplish the degree of disinfection. It is well within the ability of persons of ordinary skill in the relevant art to determine such periods of time based on the type of disinfecting solution and the degree of disinfection desired or required for the decellularized muscle matrices. For example, such periods of time may be from about 1 minute to about 4 hours, or any time range or value therebetween, such as without limitation, from about 1 minute to about 2 hours, or from about 1 minutes to about 60 minutes, or from about 5 minutes to about 2 hours, or from about 10 minutes to 2 hours, or from about 10 minutes to about 60 minutes, or from about 15 minutes to about 40 minutes, or for about 20 minutes, or for different time periods for different disinfection steps. Additionally, the (D) one or more disinfection steps may each be performed with or without suitable agitation, stirring, vacuum, pressure cycling (i.e., alternating addition and removal of pressure), sonication, perfusion, mixing, or revolutions per minute (rpm), as appropriate and determinable by persons of ordinary skill in the relevant art depending upon the apparatus and type of disinfecting solution being used.

The (D) one or more disinfection steps are each performed for a period of time sufficient to accomplish the degree of disinfection desired. It is well within the ability of persons of ordinary skill in the relevant art to determine such periods of time based on the type of disinfecting solution used and the desired resulting degree of disinfection. For example, such periods of time may be from about 1 minute to about 24 hours, or any time range or value therebetween such as without limitation, from about 1 minute to about 1 hour, or from about 10 minutes to about 30 minutes. Additionally, the (D) one or more disinfection steps may each be performed with or without suitable agitation, pressure cycling (i.e., alternating addition and removal of pressure), sonication, perfusion, stirring, mixing, or revolutions per minute (rpm), as appropriate and determinable by persons of ordinary skill in the relevant art depending upon the apparatus being used and the degree of disinfection desired.

In still other embodiments, methods for making the decellularized muscle matrices may include an optional viral inactivation step which comprises contacting the muscle tissue with one or more antiviral solutions, exposing the muscle tissue to elevated or reduced temperatures, or combinations thereof. The antiviral solutions are those capable of providing viral inactivation and/or viral load reduction and include one or more compounds selected from detergents, alcohols, acids, and bases, typically in an aqueous solvent. For example, without limitation, exposing the muscle tissue to a relatively high concentration aqueous alcohol solution (e.g., greater than 40 wt %, or greater than 50%, or greater than 60%, or greater than 70%, or greater than 80%, by weight, of at least one alcohol, based on the total weight of the aqueous alcohol solution), may provide an acceptable degree of viral inactivation.

The (E) one or more product formulation steps are generally, but not necessarily performed after most or all other processing steps as described above. The (E) one or more product formulation steps may include one or more steps of: (1) at least partial drying (e.g., by lyophilizing, air-drying, etc., up to and including less than 6% by weight moisture, based on the total weight of the matrix), (2) one or more separation steps performed by sieving, centrifuging, or both, (3) combination with one or more biocompatible carriers, (4) combination with one or more cross-linking agents (e.g., aldehydes, genipin, 1 -ethyl-3 -(3 - dimethylaminopropyl) carbodiimide (EDC, EDAC or EDCI), etc.), (5) combination with one or more additional components including without limitation, endogenous or exogenous cells, growth factors, antibiotics, pharmaceutically active agents, and other materials and substances, and (6) forming the decellularized muscle matrix into a three-dimensional matrix having a desired predetermined initial shape or form. All of the foregoing (E) product formulation steps are optional and any of them may be performed before or after other (E) product formulation steps. Furthermore, one or more (E) product formulation steps, especially those relating to combination with a carrier or other material for rehydrating a dried or lyophilized matrix, or for otherwise modifying handling characteristics such as increasing flowability for delivery by syringe, may be performed during the manufacturing or formulation processes performed to produce the decellularized muscle matrices prior to one or more of its packaging, storage, shipment or use. In some embodiments, such steps may be performed, in part or in whole, by a user (e.g., surgeon or other practitioner) at the time of use, such as immediately (e.g., within an hour) of placement on or in a recipient (patient). For example, the one or more product formulation steps (E) may comprise mixing particulate decellularized muscle matrix with one or more carriers or other additional components just prior or at the time of use, such as onsite during a medical procedure, for immediate administration to a recipient (patient).

In embodiments in which the (E) one or more product formulation steps comprise (1) one or more at least partial drying steps, each such drying step is performed for a period of time sufficient to accomplish the degree of drying desired (e.g., full or partial). It is well within the ability of persons of ordinary skill in the relevant art to determine such periods of time based on the drying technique performed, the type of apparatus employed, and the desired resulting moisture content of the final decellularized muscle matrices. For example, such periods of time may be from about 1 minute to about 36 hours, or any time range or value therebetween, such as without limitation, from about 1 minute to about 48 hours, or from about 5 minutes to about 36 hours, or from about 15 minutes to about 36 hours, or multiple time periods for multiple drying steps as, for example, in controlled rate lyophilizing. Additionally, the (1) one or more at least partial drying steps may each be performed with or without suitable agitation, stirring, vacuum, pressure cycling (i.e., alternating addition and removal of pressure), sonication, perfusion, mixing, or revolutions per minute (rpm), as appropriate and determinable by persons of ordinary skill in the relevant art depending upon the apparatus being used and the degree of drying desired.

In some embodiments, the (1) one or more at least partial drying steps may be performed in a manner which accomplishes (6) forming the decellularized muscle matrix into a three-dimensional matrix having a desired predetermined initial shape or form. For example, in some embodiments, the decellularized muscle matrix may be (3) combined with one or more biocompatible carriers and/or (5) combined with one or more additional components to form a composition, which is placed in a mold having a desired predetermined three-dimensional shape (e.g., sheet, brick, sphere, cylinder, disk, conical sections, asymmetric, irregular, etc.) and at least partially dried to produce a composition having that predetermined three-dimensional shape. In some embodiments, the decellularized muscle matrix and one or more carriers and/or additional components may be combined in the container or mold, to form the composition to be formed into a predetermined three-dimensional shape. In some embodiments, such a composition may simply be removed from the mold without drying and retain the predetermined three-dimensional shape. In some embodiments, such molded compositions having a predetermined three-dimensional shape, and which are dried or partially dried, are reshapeable into a different desired shape after rehydration by combination with one or more fluids such as a carrier, additional component, or both (e.g., one or more of PBS, saline, water, blood, plasma, PRP, BMA, etc.). Such rehydration and reshaping may occur prior to or at the time of use, or at some other desired time.

Furthermore, each of the steps of (3) combining decellularized muscle matrix with one or more biocompatible carriers and (5) combining decellularized muscle matrix with one or more additional components may be performed at any time, i.e., before, after, or both before and after, any one or more of the processing steps of the method described herein for making the decellularized muscle tissue, or even after all processing steps have been completed, before or after some period of shipment, storage, or use.

As is understood by persons of ordinary skill in the relevant art, a carrier may be biologically inert or inactive. A carrier may be biologically active, for example, in a manner which enhances the muscle-forming potential of the decellularized muscle matrices, or the carrier may provide or induce another biological activity, property or effect which may be unrelated, complementary or supplemental to the muscle-forming potential of the Decellularized muscle matrices.

Suitable biocompatible carriers may be naturally occurring or derived therefrom, or synthetic, or a combination of such materials. Generally, suitable biocompatible carriers include for example, without limitation, buffered solutions, glycerol, hyaluronate, polyethylene glycol, stearates, cellulose-derived materials (e.g., chitosan, alginates, hydroxypropyl cellulose (HPC), carboxymethyl cellulose (CMC), hydroxypropyl methyl cellulose (HPMC), etc.), and combinations thereof. Particularly suitable biocompatible carriers for use with the decellularized muscle matrices include, without limitation, hyaluronate, glycerol and buffered solutions. In some embodiments, the biocompatible carrier comprises sodium hyaluronate. In some embodiments, the biocompatible carrier comprises sodium hyaluronate and a buffered saline solution. In some embodiments, the biocompatible carrier comprises glycerol.

In some embodiments, the carrier includes at least one of an isotonic solution, a sodium chloride solution, lactated Ringer's solution, a phosphate-buffered saline solution (PBS), platelet rich plasma (PRP), bone marrow aspirate (BMA), and hyaluronic acid (HA) or a derivative thereof such as sodium hyaluronate. In embodiments, the carrier is a sodium chloride solution at a concentration of about 0.1% to about 1%. In some such embodiments, the sodium chloride solution is at a concentration of about 0.9%. In some embodiments, the carrier is a mixture of sodium hydaluronate and an aqueous solution. In some embodiments, the sodium hyaluronate has a molecular weight of from about 5.0 x 10³ to about 3.0 x 10⁶ Daltons, such as from about 6.0 x 10⁵ to about 3.0 x 10⁶ Daltons and is mixed with an aqueous solution to form a matrix-carrier mixture having a viscosity ranging from about 1000 centipoise to about 275,000 centipoise, such as from about 6,000 to about 275,000 centipoise. In some embodiments, the aqueous solution of the carrier comprising sodium hyaluronate is, for example without limitation, one or more of water, saline, phosphate buffered solution (PBS), isotonic saline, and the like.

In some embodiments, the carrier comprises thrombin. In some embodiments, the carrier comprises fibrin. In some embodiments, the carrier comprises glycerin. In some embodiments, the carrier comprises gelatin. In some embodiments, the carrier comprises collagen. In some embodiments, the carrier comprises lecithin. In some embodiments, the carrier comprises a sugar. In some embodiments, the sugar comprises a polysaccharide. In some embodiments the carrier includes a combination of two or more carrier components.

During the (E) one or more product formulation steps, the decellularized muscle matrices described herein may be mixed or otherwise (5) combined with one or more additional components for various purposes, as are well known to persons of ordinary skill in the relevant art. For example, without limitation, the decellularized muscle matrices may be mixed or otherwise combined with one or more additional components, which may or may not be biologically active, for reasons such as modifying the handling characteristics or other properties (for example without limitation, flowability, manual shapeability, moldability, degree of shape retention or memory, cohesiveness, agglomeration, flowability, porosity, etc. ), producing a particular tissue form (for example without limitation, overall (bulk) shape and size, particulate, powder, gel, paste, sponge, slurry, slivers, elongated elements, shavings, sheets, monolith, spheres, symmetric or asymmetric pieces, irregularly shaped pieces, etc.), or enhancing or adding functionality (adhesion to recipient, retention in or on recipient, adding exogenous muscle-forming potential, other tissuegenic potential, infection prevention, pH modification, increasing mass and/or available surface area, other bioactivity, etc.).

In some embodiments, where the (E) one or more product formulation steps includes (5) combining the decellularized muscle matrix with one or more additional components, the additional components may, for example without limitation, include one or more of endogenous or exogenous cells, growth factors, and collagen, as well as matrices and materials derived from autogenically, allogenically or xenogenically sourced tissues (e.g., extracellular matrix, cells, dermal-derived matrix, dermal-derived cells, amnion-derived matrix, amnion-derived cells, cartilage-derived matrix, cartilage-derived cells, muscle-derived matrix, muscle-derived cells, etc.). In some embodiments, the one or more additional components may include antibiotics, pharmaceutically active agents, ceramics, and other materials and substances.

In some embodiments, the additional component includes one or more polymers, which may be natural or synthetic. In some embodiments, the polymer is selected from the group consisting of polysaccharides, nucleic acids, carbohydrates, proteins, polypeptides, poly(a-hydroxy acids), poly(lactones), poly(amino acids), poly(anhydrides), poly(orthoesters), poly(anhydride-co-imides), poly(orthocarbonates), poly(a-hydroxy alkanoates), poly(dioxanones), poly(phosphoesters), poly(L-lactide) (PLLA), poly(D,L-lactide) (PDLLA), polyglycolide (PGA), poly(lactide-co-glycolide (PLGA), poly(L-lactide-co-D, L-lactide), poly(D,L-lactide-co-trimethylene carbonate), polyhydroxybutyrate (PHB), poly(ε-caprolactone), poly(δ-valerolactone), poly(y-butyrolactone), poly(caprolactone), polyacrylic acid, polycarboxylic acid, poly(allylamine hydrochloride), poly(diallyldimethylammonium chloride), poly(ethyleneimine), polypropylene fumarate, polyvinyl alcohol, polyvinylpyrrolidone, polyethylene, polymethylmethacrylate, carbon fibers, poly(ethylene glycol), polyethylene oxide), poly(vinyl alcohol), polyvinylpyrrolidone), poly(ethyloxazoline), poly(ethylene oxide)-co-poly(propylene oxide) block copolymers, poly(ethylene terephthalate)polyamide, and copolymers thereof.

In some embodiments, the decellularized muscle matrix is mixed with a carrier or other material to form a paste or a putty. In some embodiments, such a carrier may include a polymer, such as, without limitation, those listed above.

In some embodiments, a particulate form of decellularized muscle matrix is mixed with a carrier to form a flowable gel. In some embodiments, the mixing step is performed at a pH in the range of from about 2 to about 8. In some embodiments, the mixing step is performed at a pH in the range of about 4 to about 8. In some embodiments, the mixing step is performed at a pH below the isoelectric point of collagen.

In some embodiments, the (E) one or more product formulation steps include diluting the decellularized muscle matrix with a biocompatible solvent, forming a protein solution. In some embodiments, the protein solution has a protein concentration that is about 5% w/v or less, or about 10% w/v or less, or about 15% w/v or less or about 20% w/v or less. In some embodiments, the protein solution has a protein concentration of at least about 10 milligrams (mg) protein / milliliter (mL) protein solution and no more than about 20 mg protein / mL protein solution. In some embodiments, the protein solution has a protein concentration of from about 12 to about 18 mg protein / mL protein, or from about 14 to about 16 mg protein / mL protein. In some embodiments, the biocompatible solvent is an aqueous buffer solution. In some embodiments, the biocompatible solvent is water. In some embodiments, the protein solution is suitable for application to a large wound area. In some embodiments, the protein solution is suitable for application to the surface of a device (e.g., a nondegradable mesh, a fracture plate, a monolith, a scaffold, etc.) to increase the biocompatibility of the surface. In some embodiments, the protein solution is suitable for application in or on device (e.g., a mesh cage, a scaffold, a fabric, a gauze, a monolith, or other containment or delivery device). In some embodiments, the protein solution is suitable for application as a spray.

In some embodiments, the (E) one or more product formulating steps include (6) forming the decellularized muscle matrix into a three-dimensional matrix having a desired predetermined initial shape or form. In some embodiments, the three-dimensional matrix is formed by one or more of the processes of molding a slurry, paste, or gel, machining a sponge-like shaped decellularized muscle matrix into a different shape, using three-dimensional ("3-D") printing to deposit a flowable slurry, paste, or gel into a three-dimensional shape, laminating pieces of shaped decellularized muscle matrix, and other technologies known for use in shaping three-dimensional objects from soft or flowable materials. In some embodiments, three-dimensional decellularized muscle matrices may be provided in a lyophilized, cryopreserved, or frozen form. In some embodiments, growth factors or other soluble proteins are applied to a surface of a three-dimensional decellularized muscle matrix. In some embodiments, the growth factors or other soluble proteins may be applied to a surface of the three-dimensional decellularized muscle matrix in a pattern. In some embodiments, growth factors or other soluble proteins are placed, washed, adsorbed or infused into subsurface regions of a three-dimensional decellularized muscle matrix.

In addition to the above described steps, method for making the decellularized muscle matrices may optionally further comprise one or more sterilization steps, which may be performed before or after any of the above-described steps but which are expected to be most effective when performed after any one of the (C) one or more decellularization steps, or after any one of the (D) one or more disinfection steps, or after any of the (E) one or more product formulation steps. Any of the optional one or more sterilization steps may be performed, for example without limitation, by gamma radiation, E-beam radiation, contact with ethylene oxide, contact with supercritical carbon dioxide, exposure to heat, and combinations thereof. In some embodiments, no (D) chemical disinfection steps are performed, but rather disinfection is accomplished only by one or more sterilization steps such as application of E-Beam or gamma radiation, or heat, to the decellularized muscle matrix. In some embodiments, both (D) chemical disinfection and sterilization are performed to produce the decellularized muscle matrices.

Also, in addition to the above described steps, method for making the decellularized muscle matrices may optionally further comprise one or more delipidizing steps which remove at least a portion of lipids from the starting muscle tissue (including any other tissue types which may be present such as adipose, fascia, etc.). Some disruption of cellular membranes and removal of cells may also occur. Each of the optional one or more delipidizing steps may be performed before or after any of the above-described steps but which are expected to be most effective when performed before, after, or both before and after, any one of the (C) one or more decellularization steps, or before or after any one of the (D) one or more disinfection steps. In some embodiments, a delipidizing step comprises removing some of the lipids native to the muscle tissue. In some embodiments, a delipidizing step comprises removing most (i.e., at least 51 % (w/w), based on the total weight of the lipids naturally in the tissue) of the lipids native to the muscle tissue. In some embodiments, a delipidizing step comprises removing substantially all (i.e., at least 95 % (w/w), based on the total weight of the lipids naturally in the tissue) of the lipids native to the muscle tissue. In some embodiments, a delipidizing step disrupts cellular membranes of cells resident in the muscle tissue. In some embodiments, a delipidizing step removes cellular components from the muscle tissue. In some embodiments, one or more delipidizing steps are performed before, after, or both at least one of the (C) decellularization steps. In some embodiments, multiple delipidizing steps are performed. In some embodiments, delipidizing is not performed.

In some embodiments, delipidizing step comprises contacting the muscle tissue with a liquid so as to separate lipids from the muscle tissue. In some embodiments, delipidizing step comprises immersing the muscle tissue in the liquid. In some embodiments, delipidizing step comprises soaking the muscle tissue in the liquid. In some embodiments, a delipidizing step comprises mechanically agitating the muscle tissue in the liquid. In some embodiments, delipidizing step comprises blending the muscle tissue in the liquid. In some embodiments, blending includes a step of mixing the muscle tissue in the liquid under conditions of high shear. In some embodiments, blending includes a step of mixing the muscle tissue in the liquid using at least one rotating blade rotating at a rate in the range of from about 1,000 rpm to about 20,000 rpm. In to some embodiments, delipidizing step comprises homogenizing the muscle tissue in the liquid. In some embodiments, homogenization includes a step of mixing the muscle tissue in the liquid such that the tissue is evenly distributed throughout the liquid.

In some embodiments, the liquid is water. In some embodiments, the liquid is an organic solvent. In some embodiments, the liquid is a mixture of organic solvents. In some embodiments, the liquid is a mixture of one or more organic solvents and water. In some embodiments, the organic solvent is selected from a group consisting of a paraffin, an aromatic hydrocarbon, a cyclic hydrocarbon, a chlorinated hydrocarbon, a fluorinated hydrocarbon, a chlorinated methane, a fluorinated methane, an alcohol, an ether, a ketone, an organic acid, an aldehyde, an ester, and combinations thereof. In some embodiments, the organic solvent has one carbon atom. In some embodiments, the organic solvent has two carbon atoms. In some embodiments, the organic solvent has three carbon atoms. In some embodiments, the organic solvent has four carbon atoms. In some embodiments, the organic solvent has five carbon atoms. In some embodiments, the organic solvent has six carbon atoms.

In some embodiments, the liquid includes an organic acid. In some embodiments, the liquid includes a mineral acid. In some embodiments, the liquid includes an organic base. In some embodiments, the liquid includes a mineral base. In some embodiments, the liquid includes an organic salt. In some embodiments, the liquid contains a mineral salt.

In some embodiments, a delipidizing step comprises recovering a lipid layer by any suitable method including, without limitation, differential settling, centrifugation, filtration, decanting, or some combination thereof. In some embodiments, a delipidizing step comprises recovering the delipidized tissue by any suitable method including, without limitation, differential settling, centrifugation, filtration, decanting, or some combination thereof. In to some embodiments, a delipidization step comprises contacting the muscle tissue with a supercritical fluid (e.g., supercritical carbon dioxide), which may further include recovering the lipid by evaporation of the supercritical fluid.

In some embodiments, delipidization step is performed at a temperature of about 25°C. In some embodiments, delipidization step is performed at an ambient temperature. In some embodiments, delipidization step is performed at a temperature greater than an ambient temperature. In some embodiments, delipidization step is performed at a physiological temperature of a living mammal. In some embodiments, delipidization step is performed at a temperature of about 30°C or higher, or about 40°C or higher, or even about 50°C or higher.

In some embodiments, the delipidization step is performed under increased hydrostatic pressure. In to some embodiments, a portion of the delipidization step is performed under increased hydrostatic pressure. In some embodiments, the increased hydrostatic pressure of the delipidization step could be as high as 30,000 psi, or a fluid beyond its critical point. In some embodiments, the increased hydrostatic pressure could be achieved by performing the delipidization step in the presence of supercritical or non-supercritical gas or fluid. In some embodiments, the supercritical gas or fluid could be supercritical carbon dioxide, water or other gases or liquid beyond their critical point.

The final decellularized muscle matrices may be frozen, for example at temperatures from about -80 °C to about 0 °C, or any temperature therebetween, either before or after (F) packaging. Thus, the final decellularized muscle matrices products can be stored frozen, freeze-dried, or with a carrier. The final decellularized muscle matrices can be stored refrigerated, such as at temperature of from about 0 °C to about 8 °C, or at non-refrigerated temperatures, such as from about 8 °C to about 25 °C, or even up to about 30 °C. A suitable carrier (as described above) can be added at or near the end of process to improve cohesivity, handling and/or flexibility of the product forms. Addition of a suitable crosslinking agent may occur at any time during the above described method after the (A) one or more size and/or shape modification steps and may increase viscosity as desired and determinable by persons of ordinary skill in the relevant art.

In some exemplary embodiments, the method for making the decellularized muscle matrices comprises sequential steps performed after (A) one or more size and/or shape modification steps are performed to produce resized muscle tissue, wherein the sequential steps comprise: rinsing muscle tissue with DI water for a period of time of from about 6 hours, a first decellularization step using a first decellularizing solution comprising 0.05M TBS with 0.2% PMSF performed for about 24 hours, a second decellularization step using a second decellularizing solution comprising 2% sodium dodecyl sulfate performed for about 24 hours, a third decellularization step using a third decellularizing solution comprising 4% deoxycholate rinse performed for about 24 hours, followed by another rinsing step using DI water as the rinsing solution and performed for about 1.5 hours, a disinfection step using a disinfecting solution comprising peracetic acid performed for about 20 minutes, followed by another rinsing step using DI water and performed for about 1 hour. The foregoing exemplary method is suitable for making decellularized muscle matrices of any of the sizes and shapes of matrices described above (e.g., bulk, brick, sheet, slivers, slices, particulates, etc.).

In some exemplary embodiments, the method for making the decellularized muscle matrices comprises sequential steps performed after (A) one or more size and/or shape modification steps are performed to produce resized muscle tissue, wherein the sequential steps comprise: perform a size and shape modification step by milling a combination of thawed muscle tissue and PBS in a knife mill at about 1,000 to about 10,000 rpm for a period of from about 5 to about 600 seconds to produce particulate muscle tissue, separate and recover particulate muscle tissue having average particle size less than about 212 microns (um) using a 212-250 um sieve, perform two or more rinsing steps using DI water for about 20 minutes each to remove blood and impurities, perform a first decellularization step using a first decellularizing solution comprising 2% SDS with stirring at about 300 rpm for about 30 minutes (to permeabilize and lyse cells and remove DNA), optionally repeat the first decellularization step with a fresh quantity of the first decellularizing solution and again stirring at about 300 rpm for about 30 minutes, perform a second decellularization step using a second decellularizing solution comprising 4% sodium deoxycholate with stirring at about 300 rpm for about 30 minutes (to further lyse cells and remove myofibers), perform two or more rinsing steps using DI water for about 10 minutes each to remove residual detergents, perform a disinfection step using a disinfection solution comprising 0.2 % peracetic acid with stirring at about 300 rpm for about 20 minutes, perform three or more rinsing steps using DI water for successively longer time periods of about 5 minutes, 10 minutes and 15 minutes each to remove residual disinfectant, combine a portion of the resulting particulate decellularized muscle matrix and buffered saline in an amount sufficient to saturate the portion of matrix in a vessel (such as without limitation a glass jar, a plastic jar, a plastic syringe, or other suitable container) and lyophilize using a controlled rate freezing process. It is noted that in some embodiments, after each rinsing step, the rinsed muscle tissue may optionally be separated from the rinsing solution by sieving and recovering the rinsed muscle tissue so that fresh rising solution may be employed in a subsequent rinsing step.

Additionally, in some embodiments, one or more additional (A) size and/or shape modification steps may be performed after the chemical treatment steps are completed, but before the drying (e.g., lyophilizing) step, followed optionally by another separation step using a 212-250 um sieve to separate and recover decellularized muscle matrix having average particle size less than about 212-250 um. For example without limitation, in some embodiments, after one or more initial (A) size and/or shape modification steps, and after the chemical treatment steps including two or more (C) decellularizing steps, one disinfection step and several intermediate rinsing steps, whereby a decellularized muscle matrix in the form of slivers are formed, but before combining a portion of the sliver decellularized muscle matrix and buffered saline in a vessel and lyophilizing, the sliver decellularized muscle matrix may be subjected to one or more additional (A) size and/or shape modification steps, with or without additional size separation steps (e.g., sieving or filtering), to further reduce the size of the slivers comprising the decellularized muscle matrix. For example without limitation, such one or more additional (A) size and/or shape modification steps may comprise a first size and/or shape modification step performed by milling a combination of the sliver decellularized muscle matrix and PBS in a knife mill at about 1,000 to about 10,000 rpm (such as without limitation about 5,000 rpm) for a period of from about 5 to about 600 seconds (such as without limitation about 10 seconds), followed by a second size and/or shape modification step performed by milling a combination of the sliver decellularized muscle matrix (produced by the first size and/or shape modification step) and PBS in a knife mill at about 1,000 to about 10,000 rpm (such as without limitation about 7,500 rpm) for a period of from about 5 to about 600 seconds (such as without limitation about 5 seconds) to produce a final sliver decellularized muscle matrix ready for (E) one or more product formulation steps, such as partially or fully drying by lyophilizing.

The foregoing method for making decellularized muscle matrices may, in addition to producing particulate forms of such matrices, also be performed to produce matrices in the form of slivers when the (A) size and/or shape modification steps are performed in a manner to produce sliver-sized and -shaped muscle tissue which is then subjected to the several chemical treatment steps described above. For example, without limitation, sliver-sized and -shaped muscle tissue may be produced using a Retsch Mill for milling starting muscle tissue at about 5000 rpm for about 10 seconds. Regardless of whether the muscle tissue is in the form of particulates are slivers, the range for the speed of milling may be from about 1,000 rpm to about 10,000 rpm, or any speed therebetween, as readily determinable by persons of ordinary skill in the relevant art. Furthermore, other types of mills (such as without limitation knife mills or cryogenic mills) are also suitable to perform (A) one or more size and/or shape modification steps to produce muscle tissue in the form of smaller slivers or particulates. In some embodiments, for example, starting muscle tissue may sliced to a uniform thickness (such as without limitation about 2 mm), frozen overnight and then milled using a knife mill to produce sliver or particulate form muscle tissue which may then be subjected to several chemical treatment steps as described above.

Where sliver-sized and -shaped decellularized muscle matrix and buffered saline are combined and lyophilized in a glass jar (or other suitable container) and dried by lyophilizing, the resulting final decellularized muscle matrix is a muscle fiber sponge which may be rehydrated or not prior to use and is believed to be suitable for treatment of larger muscle defects (e.g., such as where there has been at least about 20% muscle mass lost). Where particulate or sliver-sized and -shaped decellularized muscle matrix and buffered saline are combined and dried by lyophilizing in a syringe, the resulting final decellularized muscle matrix is an aggregate of particulates or slivers which may be rehydrated prior to use by, for example without limitation, addition of buffered saline solution, to be flowable and injectable for treatment of smaller muscle defects such as muscle tears, or use for body contouring procedures. Flowable and injectable forms of the decellularized muscle matrices, whether alone or in a composition combined with a carrier or other additional component s), will pass through a syringe (i.e., cannula) of gauge 14 or higher, such as gauge 18 or higher, such as gauge 21 or higher, such as gauge 23 or higher, or such as gauge 25 or higher, without significant clogging of the syringe outlet (i.e., enabling delivery of at least 0.5 mL of matrix or composition comprising the matrix). Where particulate or sliver-sized and -shaped decellularized muscle matrix and buffered saline are combined in a syringe, without drying, the resulting final "pre-hydrated" decellularized muscle matrix is an aggregate of particulates or slivers which is ready for use as a flowable and injectable matrix for treatment of smaller muscle defects such as muscle tears, or use for body contouring procedures.

In some embodiments, a fully decellularized muscle matrix may be seeded with exogenous mesenchymal stem cells (MSCs). Such decellularized muscle matrix seeded with MSCs may successfully facilitate significant muscle regeneration compared to a decellularized muscle matrix without such additional cells. Without being limited by theory, applicants note that resident endogenous satellite cells are generally removed during decellularization and, therefore, a viable form of the decellularized muscle matrices may require adding exogenous cells.

As will be recognized by persons of ordinary skill in the relevant art, the decellularized muscle matrices described herein, as well as compositions comprising them along with one or more additional components, are suitable for use in a wide range of medical treatments and surgical procedures performed for a variety of purposes, with some forms being more suitable for some uses than others. For example, decellularized muscle matrix or a composition comprising same may be implanted into muscle tissue of a recipient for plastic and reconstructive surgery ("PRS"). Injectable forms (e.g., flowable compositions comprising particles or slivers of decellularized muscle matrix combined with a carrier such as saline, PRP, buffered saline, sodium hyaluronate, etc.) will be very useful for sports medicine related procedures as well as direct injection into or near the heart of patients in need of cardiac treatment, such as, without limitation, into or near the pericardium or other cardiac tissue.

In some embodiments, the decellularized muscle matrices are in a milled, particulate form and are injectable. Some embodiments of the decellularized muscle matrices are in a sliver form and useful as bulking agents. Some embodiments are in the form of sliver decellularized muscle matrices, while others comprise such slivers which are hydrated (e.g., combined with a biocompatible carrier) and, therefore, injectable using a syringe or other similar device.

It will be understood that the embodiments of the present invention described hereinabove are merely exemplary and that a person skilled in the art may make variations and modifications without departing from the spirit and scope of the invention. All such variations and modifications are intended to be included within the scope of the present invention.

### Examples

### Example 1 : Characterization of DNA Content

Effectiveness of the decellularization was validated by examining gross morphology and histological structure, and by assessing nuclear staining and DNA content. Nuclei were stained with 4',6-diamidino-2-phenylindole (DAPI) to determine if decellularization resulted in the ablation of cell nuclei. Removal of DNA was confirmed by extracting and purifying muscle matrices per the QIAGEN^{®} protocol (QIAamp Mini-Kit, commercially available from Quiagen having headquarters in the Netherlands and a place of business in Germantown, Maryland, U.S.A.) and quantifying DNA content of the extract using a PicoGreen^{®} dsDNA kit which is commercially available from ThermoFisher Scientific located in Waltham, Massachusetts. U.S.A.

### Example 2: Characterization of Growth Factor Content

Decellularized muscle matrix was lyophilized and 20mg was extracted using an extraction buffer for 24hr hours at 4°C on an orbital shaker. The tissue was then homogenized for 5 minutes in a tissue blender followed by centrifugation. The extract was quantified for VEGF, FGF-b and PDGF-BB using, according to manufacturers' protocols, a Raybiotech^{®} assay (commercially available from RayBiotech located in Norcross, Georgia, U.S:A.) or Luminex growth factor array (commerically available from ThermoFisher Scientific).

### Example 3: Pre-Processing of Muscle

Allograft human skeletal muscle was recovered and then stored frozen (-70°C). On the day before processing, the muscle tissue was transferred to a 4°C refrigerator to thaw overnight. Once thawed, fat and connective tissue were removed from thawed skeletal muscle. The muscle was then sliced on a drum sheer set to 2mm slice thickness. Sliced muscle tissue was divided into 30 gram portions, and each portion was placed into sealed plastic bag for storage overnight at 4 °C. Muscle tissue (240g) was placed into a Retsch Knife Mill (model GM200), and 200cc of 1XPBS was added to the chamber containing the muscle tissue. Tissue was milled at 5,000 RPM for 10 sec. Milled tissue was transferred to a 212 micron sieve to separate the tissue. De-ionized water (DI water) was added and passed through the sieve with the tissue remaining on top of the sieve. Tissue was then transferred to a metal basin containing 2L DI water. A 2L the volume was used for each rinse.

### Example 4: Processing of Muscle with Three Consecutive 4% Sodium

### Deoxycholate in DI Water Soaks

Gastrocnemius tissue (30g/200cc) was processed as described in Example 3. Blood and impurities was removed using three successive DI water rinses (20 minutes for each rinse). Tissue was then rinsed three times in 4% sodium deoxycholate in DI water for 30 min (each). In succession, this was followed by two successive DI water rinses (10 min each), disinfection in 0.2% peracetic acid (PAA) for 20 min, two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volumes were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm.

Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz lyo jar. Tissue was then frozen overnight and then freeze-dried.

DNA content of the processed tissue was then analyzed using the protocol in Example 1. DNA content was 35 ng DNA / mg tissue.

### Example 5: Processing of Muscle with One 2% Sodium Dodecyl Sulfate Soak followed by Two Consecutive 4% Sodium Deoxycholate in DI Water Soaks

Gastrocnimius tissue (30g/200cc) was processed as described in Example 3. Blood and impurities was removed using three successive DI water rinses (20 minutes for each rinse). Tissue was then rinsed once in 2% sodium deoxycholate in DI water for 30 min and then two times in 4% sodium deoxycholate in DI water for 30 min (each). In succession, this was followed by two successive DI water rinses (10 min each), disinfection in 0.2% peracetic acid (PAA) , two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve 2.5 gram of tissue was then mixed with 2.5 ml of saline, and transferred to a plastic 1 oz. lyophilization jar. Tissue was then frozen overnight and then freeze-dried.

DNA content of the processed tissue was then analyzed using the protocol in Example 1. DNA content was 17 ng DNA / mg tissue.

### Example 6: Processing of Muscle with Two 2% Sodium Dodecyl Sulfate and One 4% Sodium Deoxycholate in DI Water Soaks

Quadricep tissue (30g/200cc) was processed as described in Example 3. Blood and impurities was removed using three successive DI water rinses (20 minutes for each rinse) at 300 rpm. Tissue was then rinsed two times in 2% sodium dodecyl sulfate in DI water for 30 min each and then one time in 4% sodium deoxycholate in DI water for 30 min. In succession, this was followed by two successive DI water rinses (10 min each), disinfection in 0.2% peracetic acid (PAA) for 20 min, two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

DNA content of the processed tissue was then analyzed using the protocol in Example 1. DNA content was 5.96 ng DNA / mg tissue.

### Example 7: Processing of Muscle with Alternating DI Water and 1M NaCl Soaks

Gastrocnimius tissue (30g/200cc) processed as described in Example 3. Blood and impurities was removed by rinsing in DI water, followed by 1M NaCl, followed by DI water and followed by 1M NaCl (20 minutes for each rinse). Tissue was then rinsed once in 2% sodium dodecyl sulfate in DI water for 30 min and then two times in 4% sodium deoxycholate in DI water for 30 min. In succession, this was followed by two successive DI water rinses (10 min each) rpm, disinfection in 0.2% peracetic acid (PAA) for 20 min, two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

DNA content of the processed tissue was then analyzed using the protocol in Example 1. DNA content was 50.4 ng DNA / mg tissue.

### Example 8: Processing of Muscle with 1M NaCl Soak Before and After Decellularization

Gastrocnimius tissue (30g/200cc) was processed as described in Example 3. Blood and impurities was removed by rinsing two times in DI water followed by 1M NaCl (20 minutes for each rinse). Tissue was then rinsed once in 2% sodium dodecyl sulfate in DI water for 30 min and then two times in 4% sodium deoxycholate in DI water for 30 min. This was followed by a 20 minute rinse in 1M NaCL. In succession, this was followed by two successive DI water rinses (10 min each), disinfection in 0.2% peracetic acid (PAA) for 20 min, two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

DNA content of the processed tissue was then analyzed using the protocol in Example 1. DNA content was 37.1 ng DNA / mg tissue.

### Example 9: Processing of Muscle with Alternating DI Water and 1M NaCl Rinses followed by Three 4% Sodium Deoxycholate in DI Water Soaks

Gastrocnimius tissue (30g/200cc) was processed as described in Example 3. Blood and impurities was removed by rinsing in DI water, followed by 1M NaCl, followed by DI water and followed by 1M NaCl (20 minutes for each rinse). Tissue was then rinsed three times in 4% sodium deoxycholate in DI water for 30 min. In succession, this was followed by two successive DI water rinses (10 min each), disinfection in 0.2% peracetic acid (PAA) for 20 min, two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

DNA content of the processed tissue was then analyzed using the protocol in Example 1. DNA content was 140 ng DNA / mg tissue.

### Example 10: Processing of Muscle with 4% Sodium Deoxycholate in DI water Soaks

Rectus femoris tissue (30g/200cc) was processed as described in Example 3. Blood and impurities was removed using three successive DI water rinses (20 minutes for each rinse). Tissue was then rinsed one time in 2% sodium dodecyl sulfate in DI water for 30 min and then two times in 4% sodium deoxycholate in DI water for 30 min (each). In succession, this was followed by two successive DI water rinses (10 min each), disinfection in 0.2% peracetic acid (PAA) for 20 min, two 5 min rinses in DI water and a final rinse in DI water for 15 min. All rinse steps were at 300 rpm on a tabletop shaker In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and plastic 1 oz. lyophilization jar. Tissue was then frozen overnight and then freeze-dried.

The DNA content of the processed tissue was then analyzed using the protocol in Example 1. DNA content was 35.8 ng DNA / mg tissue.

### Example 11: Processing of Muscle with 3% Triton X-100 Soaks

Rectus femoris tissue (30g/200cc) was processed as described in Example 3. Blood and impurities was removed using three successive DI water rinses (20 minutes for each rinse). Tissue was then rinsed one time in 3% Triton X-100 for 30 min and then two times in 4% sodium deoxycholate in 25% Ethanol for 30 min each. In succession, this was followed by two successive DI water rinses (10 min each), disinfection in 0.2% peracetic acid (PAA) for 20 min and two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

The DNA content of the processed tissue was then analyzed using the protocol in Example 1. DNA content was 144 ng DNA / mg tissue.

### Example 12: Processing of Muscle with One 2% Sodium Dodecyl Sulfate Soak. Followed by Two 4% Sodium Deoxycholate in 25% Ethanol Soaks

Gracilis muscle tissue (30g/200cc) was processed as described in Example 3. Blood and impurities was removed using three successive DI water rinses (20 minutes for each rinse). Tissue was then rinsed one time in 2% sodium dodecyl sulfate in DI water for 30 min and then two times in 4% sodium deoxycholate in 25% Ethanol for 30 min each. In succession, this was followed by two successive DI water rinses (10 min each), disinfection in 0.2% peracetic acid (PAA) for 20 min and two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

The DNA content of the processed tissue was then analyzed using the protocol in Example 1. DNA content was 3.74 ng DNA / mg tissue.

### Example 13: Processing of Muscle with Three Consecutive 4% Sodium Deoxycholate in 25% Ethanol Soaks

Rectus Femoris muscle tissue (30g/200cc) was processed as described in Example 3. Blood and impurities was removed using three successive DI water rinses (20 minutes for each rinse). Tissue was then rinsed three times in 4% sodium deoxycholate in 25% Ethanol for 30 min each. In succession, this was followed by two successive DI water rinses (10 min each), disinfection in 0.2% peracetic acid (PAA) for 20 min and two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm.

Following milling, tissue was transferred to a 212 micron sieve to separate the tissue. 2.5 gram of tissue was then mixed with 2.5 ml of saline, and transferred to a plastic 1 oz. lyophilization jar. Tissue was then frozen overnight and then freeze-dried.

The DNA content of the processed tissue was then analyzed using the protocol in Example 1. DNA content was 80 ng DNA / mg tissue.

### Example 14: Processing of Muscle with Two consecutive 3% Triton soaks followed by 4% Sodium Deoxycholate in DI Water Soaks

Rectus femoris tissue (30g/200cc) was processed as described in Example 3. Blood and impurities was removed using three successive DI water rinses (20 minutes for each rinse). Tissue was then rinsed two times in 3% Triton X-100 for 30 min and then one time in 4% sodium deoxycholate in DI water for 30 min each. In succession, this was followed by two successive DI water rinses (10 min each), disinfection in 0.2% peracetic acid (PAA) for 20 min and two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried.

The DNA content of the processed tissue was then analyzed using the protocol in Example 1. DNA content was 328 ng DNA / mg tissue.

### Example 15: Processing of Muscle into a Sponge

Allograft human skeletal muscle was recovered and then stored frozen (-70°C). On the day before processing, the muscle tissue was transferred to a 4°C refrigerator to thaw overnight. Once thawed, fat and connective tissue were removed from thawed skeletal muscle. The muscle was then sliced on a drum sheer set to 2mm slice thickness. Sliced muscle tissue was divided into 30 gram portions, and each portion was placed into sealed plastic bag for storage overnight at 4C.

Muscle tissue (30g) was placed into a Retsch Knife Mill (model GM200), and 200cc of 1XPBS was added to the chamber containing the muscle tissue. Tissue was milled at 5,000 RPM for 10 sec. Milled tissue was transferred to a 212 micron sieve to separate the tissue. De-ionized water (DI water) was added and passed through the sieve with the tissue remaining on top of the sieve. Tissue was then transferred to a metal basin containing 2L DI water. A 2L the volume was used for each rinse.

Blood and impurities was removed using three successive DI water rinses (20 minutes for each rinse) at 300 rpm. Tissue was then rinsed two times in 2% sodium dodecyl sulfate in DI water for 30 min each (to permeabilize & lyse cells and remove DNA), with the SDS being replenished with fresh solution between the first and second SDS rinse. The muscle matrix was then rinsed one time in 4% sodium deoxycholate in DI water for 30 min to further lyse cells and remove myofibers.

(Sodium deoxycholate is a less harsh detergent which further permeabilizes & lyse cells and removes myofibers and DNA.) In succession, this was followed by two successive DI water rinses (10 min each), disinfection in 0.2% peracetic acid (PAA) for 20 min, two 5 min rinses in DI water and a final rinse in DI water for 15 min. In between all rinse steps, the tissue was sieved and the liquid was replenished. All rinse and chemical treatment steps occurred at 300 rpm on a tabletop shaker. All rinse volume were 2L for each step. All rinse volume were 2L for each step.

A post-processing milling step was then performed, whereby the tissue was milled in the Retsch Mill (Model GM200) by adding the processed tissue to the milling chamber and adding 200cc of IX PBS. Tissue was then milled for 10 seconds at 5000 rpm followed by 5 seconds at 7,500 rpm. Following milling, tissue was transferred to a 212 micron sieve to separate the tissue using DI water, with the tissue remaining on top of the sieve. 2.5 gram of tissue was then mixed with 2.5 ml of saline, and transferred to a plastic 1 oz. lyophilization jar. Tissue was then frozen overnight and then freeze-dried to form a sponge-like structure.

### Example 16: Processing of Muscle for Injection

Quadricep tissue was processed as described in Example 15. The resulting sponge-like structure was cut into 0.16g pieces and transferred to a 3cc syringe. Saline (1 cc) was transferred to a separate syringes. Using luer adapters, the two syringes were connected and the saline was passed from its syringe into the one containing the sponge-like tissue. Tissue is hydrated quickly, and fully hydrated within 5 min. After 5 minutes, the tissue was then mixed back and forth between the two syringes 10 times. An emulsifier having apertures 0.8mm in diameter (available from Tulip Medical Products located in San Diego, California, U.S.A.) was used to breakdown and homogenize the tissue further for injection, and the tissue was homogenized back and forth 10 times. A 18-gauge needle was then attached to the syringe, and the hydrated tissue was able to be expelled through the syringe.

### Example 17: Growth Factor Content in Non-Processed Muscle

The growth factor content of native, non-processed muscle was quantified using the methods described in Example 2 for the Luminex^{®} assay (ThermoFisher Scientific) and the results are reported in Table 1 below. All values are in pg/ml.

**Table 1**

| **Muscle** | **VEGF-A (pg/mL)** | **FGF basic (pg/mL)** |
|---|---|---|
| **Quadriceps** | 359.38 | >482.16↑ |
| **Gastroenemius** | 177.2 | >482.16↑ |
| **Sartorius** | 166.49 | >482 16↑ |
| **Rectus Femoris** | 745.42 | >482.16↑ |
| **Gracilis** | 138.92 | >482.16↑ |
| **Semi Tendonous** | 228.78 | 375.97 |

↑ indicates above the limit of detection.

### Example 18: Growth Factor Content - Processed Quadriceps

Quadriceps muscle was processed using the method described in Example 6, and the growth factor content of the processed quadriceps tissue was then quantified using the method of Example 2 for the Luminex^{®} assay (ThermoFisher Scientific) and the results are reported in Table 2 below.

**Table 2**

| **Muscle** | **VEGF-A (pg/mL)** | **FGF basic (pg/mL)** |
|---|---|---|
| **Quadriceps** | 5.52 | 7.01 |

### Example 19: Growth Factor Content - Processed Gastrocnemius / Rectus Femoris

Gastrocnemius and Rectus Femoris muscle were processed together using the method described in Example 6, and the growth factor content was then quantified using the method of Example 2 for the Luminex^{®} assay (ThermoFisher Scientific) and the results are reported in Table 3 below.

**Table 3**

| **Muscle** | **VEGF-A (pg/mL)** | **FGF basic (pg/mL)** |
|---|---|---|
| Gastrocnemius / Rectus Femoris | 12.56 | 2.51 |

### Example 20: Growth Factor Content of Processed Muscle Tissue

Allograft muscle (Gastrocnemius, Gracillis, Semi-tendinosis, Quadricep and Peruneus Longus) from 6 donors was recovered and processed per Example 6. Growth factor content (FGF-basic and VEGF) of the the final, processed muscle tissue was analyzed using the method of Example 2 and a Luminex^{®} array (ThermoFisher Scientific) and the results are reported in Table 4 below.

**Table 4**

| **Donor** | **Condition** | **FGF-b (ng/g)** | **VFG F-A (ng/g)** |
|---|---|---|---|
| 1 | Processed | 0,6112 | 0,3944 |
| 2 | Processed | 0.5612 | 1.022 |
| 3 | Processed | 0.4392 | 0.4876 |
| 4 | Processed | 0.3852 | 0.7852 |
| 5 | Processed | 0.8704 | 1.3488 |
| 6 | Processed | 0.5992 | 1.3384 |

### Example 21: Growth Factor Content of Processed Muscle Tissue

Allograft muscle (Quadricep, Gastrocnemius, Sartorius, Rectus Femoris, Gracilis, and Semi tendinosis) from 6 donors was recovered and processed per Example 6. Growth factor content (FGF-basic and VEGF) of the final, processed muscle tissue was analyzed using the methods of Example 2 and a Raybiotech^{®} array (RayBiotech) and the results are reported in Table 5 below.

**Table 5**

| **Donor** | **Condition** | **FGF-b (ng/g)** | **VEGF-A (ng/g)** |
|---|---|---|---|
| 1 | Processed | 3 554841 | 2 341968 |
| 1 | Processed | 2.225117 | 0.62541 |
| 3 | Processed | 1.709767 | 3 191299 |
| 4 | Processed | 1.512073 | 3.06146 |
| 5 | Processed | 7.257738 | 4.363954 |
| 6 | Processed | 1 817371 | 0.65771 |

### Example 22: Animal Study

*Particulate* muscle was processed into sponges per Example 15, rehydrated with saline and implanted into a surgically created muscle defect in the hind leg of an athymic rat.

*Particulate* muscle was processed into injectable form per Example 16 and A) delivered via intramuscular injection into the hind leg muscle of an athymic rat or B) administered on the flanks of athymic rats. A total of 10 athymic rats were tested. At day 21 the rats will be anesthetized and the implant (and surrounding tissue) will be assessed for gross and histologic properties, including volume retention and immunogenic/antigenic responses.

### Example 23: Comparison of un-processed vs. processed muscle tissue

Allograft muscle (Quadricep, Gastrocnemius, Sartorius, Rectus Femoris, Gracilis, and Semi tendinosis) from 6 donors was recovered and processed per Example 6. Histological analysis was performed on native, un-processed tissue and the processed tissue from the same s=six donors. H&E staining demonstrated removal of myofibers in processed tissue. DAPI staining demonstrated removal of DNA in processed tissues. Immunohistochemical (IHC) staining demonstrated retention of collagen IV, fibronectin, laminin, glycosaminoglycan (GAG) and ECM (extra-cellular matrix) in processed tissue relative to native, un-processed tissues. IHC staining also demonstrated removal of alpha actin, desmin, myosin and muscle markers in processed tissue relative to native, un-processed tissue.

Muscle matrix samples were stained for H&E, DAPI, and muscle specific markers (myosin, desmin, alpha-actin) before and after processing to assess the extent of decellularization. The H&E and DAPI stains demonstrated that myofibers and DNA were removed, respectively. The muscle specific markers (myosin, desmin, alpha-actin) revealed that muscle specific marker was removed as well.

Paraffin-embedded sections of whole muscle samples were compared to decellularized muscle using Masson's trichrome stain.

IHC stains for ECM proteins (Collagen IV, fibronectin, safranin-o and laminin) demonstrated that the basement membranes remained intact after decellularization.

### Example 24: Handling assessment for 3D sponges

Decellularized muscle matrix was processed per Example 15 and lyophilized into a 27mm in diameter sponge. Handling assessment showed the resulting sponge was elastic, rehydrated in Saline within 5-10 seconds and remained cohesive and moldable.

### Example 25: Handling assessment for Injectable Form

Decellularized muscle matrix was processed per Examples 15 and 16 to create an injectable version. For this, 0.160g of lyophilized decellularized muscle matrix was aliquoted into a 3cc syringe and Icc saline was aliquoted into a separate syringe. The muscle matrix was reconstituted via a luer-lock for 5 minutes. The tissue was then mixed through the two syringes ten times. A 0.8mm in diameter emulsification device (Tulip Medical Products) was then connected and the tissue was homogenized back and forth ten times. An 18 gauge needle was then attached to the syringe and the hydrated muscle sliver was able to be extruded.

### Example 26: Removal of Myofibers

Muscle matrix samples were stained for H&E, before and after processing to assess the extent of decellularization and myofiber removal. The H&E stains demonstrated that myofibers were removed, as indicated by the hollow structures void of the pink (myofiber) stain while retaining the structural integrity of the surrounding basement membranes.

### Example 27: Processing of Muscle with Two 2% Sodium Dodecyl Sulfate and One 4% Sodium Deoxycholate in DI Water Soaks using an Increased Capacity Mixing Chamber

Allograft human skeletal muscle is recovered and then stored frozen (-70°C). On the day before processing, the muscle tissue is transferred to a 4°C refrigerator to thaw overnight. Once thawed, fat and connective tissue is removed from thawed skeletal muscle. The muscle is then sliced on a slicing device which was set to 2mm slice thickness. Sliced muscle tissue is divided into 30 gram portions, and each portion is placed into sealed plastic bag for storage overnight at 4C.

Muscle tissue (120g) is placed into a Retsch Knife Mill (model GM300), and 800cc of DI water is added to the chamber containing the muscle tissue. Tissue is milled one or more times at 3,500 RPM for up to 60 seconds. Milled tissue is transferred to a 212 micron sieve to separate the tissue, and then to a metal basin containing 2L DI water, with the tissue remaining on the top of the sieve. Blood and impurities are removed using successive DI water rinses. Tissue is then rinsed two times in 2% sodium dodecyl sulfate (SDS) in DI water for 30 min each (to permeabilize & lyse cells and remove DNA), with the SDS being replenished with fresh solution between the first and second SDS rinse. The muscle matrix is then rinsed one time in 4% sodium deoxycholate in DI water for 30 min to further lyse cells and remove myofibers. (Sodium deoxycholate is a less harsh detergent which further permeabilizes & lyse cells and removes myofibers and DNA.) This is followed by DI water rinses.

Disinfection of the tissue is then performed in 1L of 0.2% peracetic acid (PAA) for 20 min. Residual PAA is removed through a series of rinses in DI water. In between all rinse steps, the tissue is sieved and the liquid is replenished. All rinse and chemical treatment steps occur at 300 rpm. All rinse volumes are 2L for each step.

A post-processing milling step is then performed, whereby the tissue is milled in the Retsch Mill (Model GM300) one or more times by adding the processed tissue to the milling chamber and adding 800cc of DI water. Tissue is then milled for up to 60 seconds at 3,500 rpm. Following milling, tissue is transferred to a 212 micron sieve to separate the tissue. 2.5 gram of tissue is then mixed with 2.5 ml of saline, and transferred to a plastic 1 oz. lyophilization jar. Tissue is then frozen overnight and then freeze-dried to form a decellularized muscle matrix having sponge-like structure (i.e.,"Decellularized Muscle Matrix Sponge").

### Example 28: Processing of Muscle for Injection

Quadricep tissue is processed as described in Example 27. The resulting Decellularized Muscle Matrix Sponge from the process of Example 27 is cut into 0.16g pieces and transferred to a 3cc syringe. Saline (1 cc) is transferred to a separate syringe. Using luer adapters, the two syringes are connected and the saline is passed from its syringe into the one containing the Decellularized Muscle Matrix Sponge. The Decellularized Muscle Matrix Sponge is hydrated quickly, and fully hydrated within 5 min. After 5 minutes, the hydrated Decellularized Muscle Matrix Sponge is then mixed back and forth between the two syringes 10 times. An emulsifier 0.5-0.8mm emulsifier device (Tulip Medical Products) is then used to breakdown and homogenize the hydrated Decellularized Muscle Matrix Sponge further for injection, and the hydrated Decellularized Muscle Matrix Sponge is homogenized back and forth 10 times. An 18-gauge needle is then attached to the syringe, and the hydrated Decellularized Muscle Matrix Sponge is able to be expelled through the syringe.

### Example 29: Preparation of Muscle Matrix Samples for a Pre-Clinical Rat Study and Histopathological Analysis

Allograft human skeletal muscle was recovered and processed as described in Example 27. Endogenous blood vessels and connective tissue were removed from the muscle atop the sieve after each processing step.

The samples were evaluated in an athymic rat model and the extent of muscle regeneration, number of central nuclei and blood vessel formation were quantified in Table 6 below according to the Scale also provided below.

**Table 6**

| **Clinical Observation** | **Day 0** | **Week 4** | **Week 8** |
|---|---|---|---|
| Extent of new muscle regeneration* | + | ++ | ++++ |
| Number of Central Nuclei | + | ++ | ++++ |
| Blood vessel formation | + | +++ | +++ |

| | | | |
|---|---|---|---|
| * Assessed by histological stains: H&E, Masson's Trichrome, muscle specific markers | | | |

| Scale | |
|---|---|
| + | 0 |
| ++ | 25% |
| +++ | 50% |
| ++++ | 75% |
| +++++ | 100% |

### Example 30: Preparation of Muscle Matrix Sponge Samples for a Pre-Clinical Rat VML Study

Allograft human skeletal muscle was recovered, sliced with a slicing device which was set to a 2mm thickness and processed as described in Example 27. Endogenous blood vessels and connective tissue were retained in the samples.

### Example 31: VML Animal Study

Particulate muscle is processed into sponges per Example 27, rehydrated with saline and implanted into a surgically created VML muscle defect (see Example 32 below), created within the range of 15-30% muscle mass removal, in the tibialis anterior (TA) muscle of rats.

Next, the rats perform daily exercise on a treadmill beginning 2-3 days post-op. The duration of the study is 2, 8, 12 and 16 weeks, and the implant (and surrounding tissue) are assessed for gross and histologic properties, including volume retention (assessed by MRI and ultrasound) , immunogenic/antigenic responses and muscle regeneration. The functional restoration is evaluated via gait analysis, dynamic weight bearing and isometric tetanic force evaluation.

### Example 32: Volumetric Muscle Loss Defect Creation Protocol

A longitudinal incision is made along the lateral aspect of the lower left leg over the tibialis anterior (TA) muscle, and the TA muscle is exposed and separated from the tibia. A central zone defect is created in the TA muscle. The defect is approximately 7 mm wide and is the length of the muscle. The test article is applied directly over the defect and secured with overlying tissue. The fascia and skin are then closed using appropriate sutures.

In the following, some aspects of the present disclosure are summarized:
1. A composition comprising a decellularized muscle matrix which provides endogenous muscle-forming potential for muscle repair and regeneration and was derived from unprocessed muscle tissue containing endogenous cytokines and endogenous extracellular proteins, wherein the decellularized muscle matrix retains one or more of the endogenous growth factors, endogenous cytokines, and endogenous extracellular proteins.
2. The composition of Aspect 1, wherein the one or more endogenous growth factors are selected from one or more of: vascular endothelial growth factor (VEGF), fibroblast growth factor - basic (FGF-b), and insulin growth factor (IGF).
3. The composition of Aspect 1, wherein the endogenous extracellular components include collagen IV, laminin, and fibronectin.
4. The composition of Aspect 1, wherein the unprocessed muscle tissue contained an initial quantity of endogenous immunogenic components which included myofibers and deoxyribonucleic acid (DNA), and wherein the decellularized muscle matrix contains less than the initial quantity of endogenous immunogenic components.
5. The composition of Aspect 1, wherein, wherein the decellularized muscle tissue matrix is fully decellularized.
6. The composition of Aspect 1, wherein the decellularized muscle tissue matrix has a physical form selected from: strips, chunks, bulk, brick, pieces, elongated elements, sheets, slivers, slices, elongated elements, shavings, particulates, homogenate, and combinations thereof.
7. The composition of Aspect 6, wherein the physical form of the decellularized muscle matrix is slivers, elongated elements, or a combination thereof.
8. The composition of Aspect 1, wherein, wherein the decellularized muscle tissue matrix is in dried form.
9. The composition of Aspect 1, wherein the composition is dried or partially dried and is shapable or reshapable into a different shape after rehydration by combination with one or more fluids such as a carrier, additional component, or both.
10. The composition of Aspect 1, wherein, wherein the decellularized muscle tissue matrix is hydrated and moldable.
11. The composition of Aspect 1, wherein the composition has been formed into a predetermined three-dimensional shape.
12. The composition of Aspect 11, wherein the three-dimensional shape has been formed using a mold.
13. The composition of Aspect 1, further comprising a biocompatible carrier.
14. The composition of Aspect 13, wherein the biocompatible carrier is selected from: buffered solutions, glycerol, hyaluronate, polyethylene glycol, stearates, cellulose-derived materials, carboxymethyl cellulose (CMC), hydroxypropyl methyl cellulose (HPMC), and combinations thereof.
15. The composition of Aspect 13, wherein the composition is flowable and capable of injection through a syringe.
16. The composition of Aspect 1, further comprising one or more additional components.
17. The composition of Aspect 16, wherein the one or more additional components are selected from: polymers, ceramics, materials derived from other tissue types, extracellular matrix material derived from one or more tissue types, exogenous growth factors, cells, antibiotics, and combinations thereof.
18. The composition of Aspect 1, further comprising one or more biocompatible carriers and one or more additional components.
19. The composition of Aspect 1, further comprising one or more additional components selected from: endogenous or exogenous cells, growth factors, antibiotics, pharmaceutically active agents, and combinations thereof.
20. The composition of Aspect 1, wherein the unprocessed muscle tissue was recovered from one or more donors, at least one of which was human or non-human species of animal.
22. A method for preparing a decellularized muscle tissue matrix comprising the steps of:
   (A) optionally, modifying size, shape, or both, of muscle tissue;
   (B) rinsing muscle tissue, before, after, or both before and after any other step;
   (C) performing one or more decellularization soaks; and
   (D) optionally, disinfecting muscle tissue.
23. The method of Aspect 22, wherein the step of (A) modifying size, shape, or both, of muscle tissue comprises one or more of cutting, slicing, grating, grinding, mincing, and homogenizing unprocessed muscle tissue.
24. The method of Aspect 22, wherein the step of (B) rinsing muscle tissue comprises contacting the muscle tissue, for a period of rinsing time, with at least one rinsing solution, before, after, or both before and after, any (C) decellularizing and (D) disinfecting steps.
25. The method of Aspect 22, wherein the step of (C) performing one or more decellularization soaks comprises contacting the muscle tissue for a period of decellularizing time with at least one decellularizing solution comprising at least one detergent.
26. The method of Aspect 25, wherein all decellularizing solutions used substantially lack pepsin and pepsin derivatives.
27. The method of Aspect 25, wherein the step of (C) performing one or more decellularization soaks comprises three separate decellularization soaks, a first of which includes contacting the muscle tissue with a first decellularizing solution comprising tris-buffered saline and phenyl methyl sulfonyl fluoride (PMSF), a second of which includes contacting the muscle tissue with a second decellularizing solution comprising sodium dodecyl sulfate (SDS), and a third of which includes contacting the muscle tissue with a third decellularizing solution comprising sodium deoxycholate.
28. The method of Aspect 25, wherein the step of (C) performing one or more decellularization soaks comprises two separate decellularization soaks, a first of which includes contacting the muscle tissue with a first decellularizing solution comprising sodium dodecyl sulfate (SDS), and a second of which includes contacting the muscle tissue with a second decellularizing solution comprising sodium deoxycholate.
29. The method of Aspect 22, wherein the step of (D) disinfecting muscle tissue comprises contacting the muscle tissue, for a period of disinfecting time, with a disinfection solution comprising at least one disinfectant.
30. The method of Aspect 22, wherein the step of (D) disinfecting muscle tissue comprises contacting the muscle tissue for a first period of disinfecting time, with a first disinfection solution comprising peracetic acid, and contacting the muscle tissue for a second period of disinfecting time, with a second disinfection solution comprising one or more antibiotics.
31. The method of Aspect 22, further comprising the step of delipidizing the muscle tissue, for a period of delipidizing time, with at least one delipidizing liquid, before, after, or both before and after, any (C) decellularizing and (D) disinfecting steps.
32. The method of Aspect 22, wherein the delipdizing liquid comprises one or more organic solvents.
33. The method of Aspect 22, further comprising the steps of:
   (E) formulating a product comprising the decellularized muscle tissue matrix; and
   (F) optionally, packaging the decellularized muscle matrix or product comprising the decellularized muscle tissue matrix.
34. A method for providing muscle-forming potential to a recipient comprising combining the composition of Aspect 1, which may be at least partially dried or not, with one or more carriers, one or more additional component, or both, during a medical procedure in which the composition will be implanted into the recipient.
35. The method of Aspect 34, wherein the one or more carriers are selected from: platelet rich plasma, bone marrow aspirate, hyaluronate, amniotic fluid, lipoaspirate and combinations thereof.
36. The method of Aspect 34, wherein the one or more additional components are selected from: polymers, ceramics, materials derived from other tissue types, extracellular matrix material derived from one or more tissue types, exogenous growth factors, cells, antibiotics, and combinations thereof.
37. A method for repair and regeneration of muscle tissue of a recipient, the method comprising implanting the composition of Aspect 1 in or on the recipient.
38. The method of Aspect 37, wherein the decellularized muscle matrix supports differentiation and renewal of muscle progenitor cells.
39. The method of Aspect 37, wherein the recipient has a condition which is treatable by repair and regeneration of muscle tissue and the condition is at least one of: volumetric muscle loss, muscle atrophy, degenerative disease, cardiac disease, cardiac damage, sports injuries, and other injuries.
40. A method for cosmetic treatment of a recipient, the method comprising implanting the composition of Aspect 1 in or on the recipient.
41. A method for nerve re-innervation in a recipient, the method comprising implanting the composition of Aspect 1 in or on the recipient.
42. A method for re-vascularization in a recipient, the method comprising implanting the composition of Aspect 1 in or on the recipient.

## Claims

1. A composition comprising a decellularized muscle matrix which is at least partially decellularized and derived from unprocessed muscle tissue having endogenous muscle-forming potential and containing endogenous growth factors, endogenous cytokines and endogenous extracellular proteins, wherein the decellularized muscle matrix retains at least a portion of the endogenous muscle-forming potential which promotes muscle repair and regeneration and at least a portion of one or more of the endogenous growth factors, the endogenous cytokines, and the endogenous extracellular proteins from the unprocessed muscle tissue.

2. The composition of Claim 1,
wherein either (A) the one or more endogenous growth factors are selected from one or more of: vascular endothelial growth factor (VEGF), fibroblast growth factor - basic (FGF-b), and insulin growth factor (IGF), (B) the endogenous extracellular components include collagen IV, laminin, and fibronectin, or both (A) and (B).

3. The composition of Claim 1 or 2,
wherein the unprocessed muscle tissue contained an initial quantity of endogenous immunogenic components which included myofibers and deoxyribonucleic acid (DNA), and wherein the decellularized muscle matrix contains less than the initial quantity of endogenous immunogenic components.

4. The composition of one of Claims 1 to 3,
wherein the decellularized muscle tissue matrix has a physical form selected from: strips, chunks, bulk, brick, pieces, sheets, slivers, slices, elongated elements, shavings, particulates, homogenate, and combinations thereof.

5. The composition of one of Claims 1 to 4,
wherein the composition is dried or partially dried and is shapable or reshapable into a different shape after rehydration by combination with one or more fluids such as a carrier, additional component, or both.

6. The composition of one of Claims 1 to 5,
wherein the decellularized muscle tissue matrix is hydrated and moldable.

7. The composition of one of Claims 1 to 6,
wherein the composition has been formed into a predetermined three-dimensional shape, using a mold or not.

8. The composition of one of Claims 1 to 7,
further comprising a carrier, one or more additional compounds, or both.

9. The composition of Claim 8,
wherein the carrier is selected from: an isotonic solution, a sodium chloride solution, lactated Ringer's solution, a phosphate-buffered saline solution (PBS), blood, plasma, platelet rich plasma (PRP), bone marrow aspirate (BMA), hyaluronic acid (HA), a derivative of HA, buffered solutions, glycerol, hyaluronate, polyethylene glycol, stearates, cellulose-derived materials, carboxymethyl cellulose (CMC), hydroxypropyl methyl cellulose (HPMC), and combinations thereof.

10. The composition of one of Claims 1 to 8,
further comprising a carrier and being capable of injection through a syringe.

11. A method for providing muscle-forming potential to a recipient comprising implanting the composition of one of Claims 1 to 10, in or on the recipient, during a procedure for treatment of volumetric muscle loss, muscle atrophy, muscle damage or injury, degenerative disease, cardiac condition, sports injury, and combinations thereof, wherein the composition optionally further comprises one or more carriers, one or more additional component, or both.

12. A method for repair and regeneration of muscle tissue of a recipient, the method comprising implanting the composition of one of Claims 1 to 10 in or on the recipient, during a procedure for treatment of volumetric muscle loss, muscle atrophy, muscle damage or injury, degenerative disease, cardiac condition, sports injury, and combinations thereof, wherein the composition optionally further comprises one or more carriers, one or more additional component, or both.

13. A method for cosmetic or reconstructive treatment of a recipient, the method comprising implanting the composition of one of Claims 1 to 10 in or on the recipient, during a procedure comprising body feature contouring, sculpting, bulking or volume restoration and combinations thereof, wherein the composition optionally further comprises one or more carriers, one or more additional component, or both.

14. A method for nerve re-innervation in a recipient, the method comprising implanting the composition of one of Claims 1 to 10 in or on the recipient, during a procedure for muscle repair and regeneration wherein the composition is a scaffold and provides or supports one or more of:
mechanical stability, differentiation and renewal of muscle progenitor cells (satellite cells), muscle repair, and muscle tissue generation, wherein the composition optionally further comprises one or more carriers, one or more additional component, or both. wherein the composition optionally further comprises one or more carriers, one or more additional component, or both.

15. A method for re-vascularization in a recipient, the method comprising implanting the composition of one of Claims 1 to 10 in or on the recipient, during a procedure for muscle repair and regeneration wherein the composition is a scaffold and provides or supports one or more of:
mechanical stability, differentiation and renewal of muscle progenitor cells (satellite cells), muscle repair, and muscle tissue generation, wherein the composition optionally further comprises one or more carriers, one or more additional component, or both.
